# EUROPEAN PATENT APPLICATION

(11) **EP 1 746 106 A2**
(43) Date of publication of application: **24.01.2007**
(21) Application number: 06118875.1
(22) Date of filing: 27.04.2001
(51) Int. Cl.: C07K 14/705, C12N 15/12, C12N 15/62, C07K 19/00, A61P 35/00

(54) **Use of TACI as an anti-tumor agent**

(30) Priority: 27.04.2000 US 199946 P
(62) Divisional of application: 01927474.5
(71) Applicant: Biogen Idec MA Inc., Cambridge, Massachusetts 02142 (US); Apoxis SA, 1066 Epalinges (CH)
(72) Inventor: Ambrose, Christine, Reading, MA 01867 (US); Thompson, Jeffrey, Stoneham, MA 02180 (US); Schneider, Pascal, 1066 Epalinges (CH); Rennert, Paul, Millis, MA 02054 (US)
(74) Representative: Pohlman, Sandra M.

(57) **Abstract**

A method of treating a mammal for a condition associated with undesired cell proliferation comprising administering to said mammal an effective amount an effective amount of a TACI reagent, wherein said reagent extends mean survival time of said mammal by about 10 % or more as compared to the absence of administering the TACI reagent.

## Description

### FIELD OF THE INVENTION

The present invention relates generally to methods of treating a mammal for a condition associated with undesired cell proliferation, including cancer.

### BACKGROUND OF THE INVENTION

Members of the tumor-necrosis factor (TNF) family of cytokines are involved in an ever-expanding array of critical biological functions. Each member of the TNF family acts by binding to one or more members of a parallel family of receptor proteins, namely, the TNF receptor family of proteins. TNF receptors, which in turn, signal intracellularly to induce a wide range of physiological or developmental responses. Many of the TNF receptor signals influence cell fate, and often trigger terminal cellular differentiation. Examples of cellular differentiation include proliferation, maturation, migration, and death.

In U.S. patent number 5,969,102, incorporated by reference herein, TACI (Transmembrane Activator CAML Interactor protein), a novel TNF family member receptor protein, is described. The corresponding TNF family member ligand to TACI, however, was not known.

The present invention discloses that the TNF family member APRIL, which is described in applicants co-pending international application PCT/US98/19191, is a ligand to TACI. It is also a discovery of the present invention that TACI reagents are particularly useful in treating a mammal for a condition associated with undesired cell proliferation, including for example, cancer.

### SUMMARY OF THE INVENTION

The present invention is directed to a method of treating a mammal for a condition associated with undesired cell proliferation comprising administering to a mammal an effective amount of a TACI reagent. Conditions associated with undesired cell proliferation include but are not limited to cancer and specifically renal cell cancer, Kaposi's sarcoma, prostate cancer, breast cancer, sarcoma, ovarian carcinoma, rectal cancer, throat cancer, melanoma, colon cancer, bladder cancer, mastocytoma, lung cancer, mammary adenocarcinoma, pharyngeal squamous cell carcinoma, gastrointestinal cancer, and stomach cancer.

In preferred embodiments, the present invention relates to methods of treating a mammal for conditions associated with undesired cell proliferation wherein such cell proliferation is associated with solid tumors. In particular, such solid tumor cancerous conditions include tumors of the prostate, lung, breast, colorectal, bladder, endometrium, ovary, oropharynx/larynx, cervix, stomach, pancreas, and the brain (and central nervous system).

Also contemplated are methods for reducing the size of a tumor located on or in a mammal comprising administering to said mammal an effective amount of a TACI reagent. In preferred embodiments, the tumor is a solid tumor.

The methods of the present invention include the use of a fusion protein comprising at least two segments, wherein a first segment comprises a substantially pure TACI protein or polypeptide fragment thereof, and a second segment comprises an immunoglobulin polypeptide. The immunoglobulin polypeptide is preferably a human IgG Fc domain.

It is to be understood from the foregoing general description and the following detailed description are exemplary and explanatory, and are intended to provide further explanation of the invention claimed.

### BRIEF DESCRIPTION OF THE DRAWINGS

The accompanying drawings are included to provide a further understanding of the invention, and are incorporated in, and constitute a part of this specification, illustrate several embodiments of the invention, and together with the description serve to explain the principles of the invention.

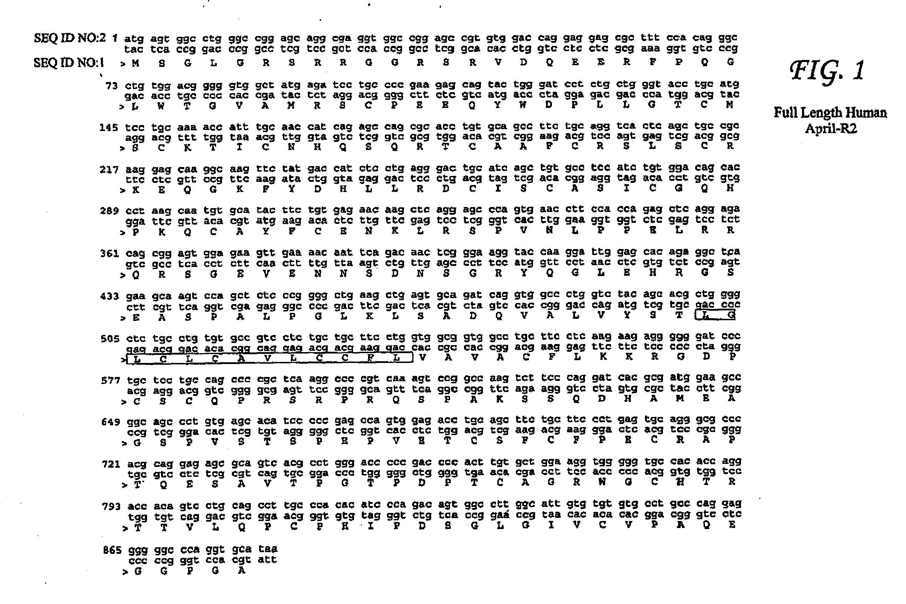

**Figure 1** is a schematic representation of the nucleic acid sequence (SEQ ID NO:2) of a cDNA for human TACI and its derived amino acid sequence (SEQ ID NO:1) as mapped in vector pCA336.

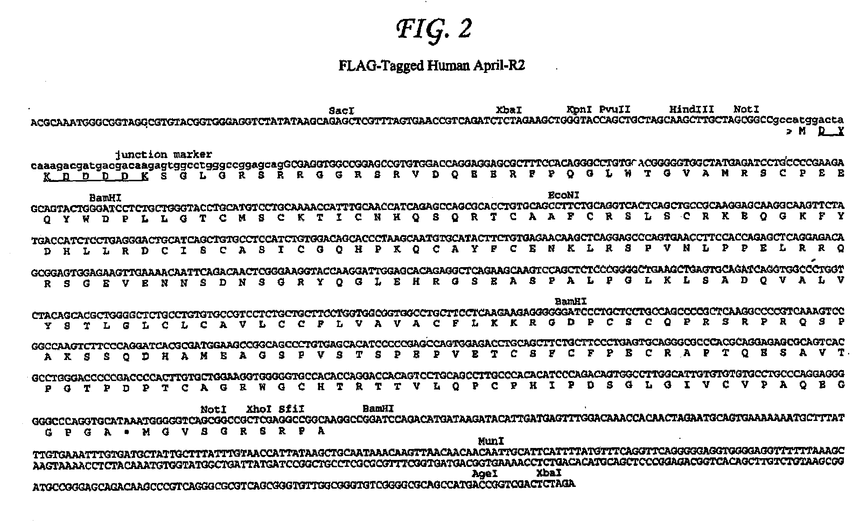

**Figure 2** is a schematic representation of the nucleic acid sequence insert in pJST552 encoding N-terminus FLAG-tagged human full length TACI, and its derived amino acid sequence.

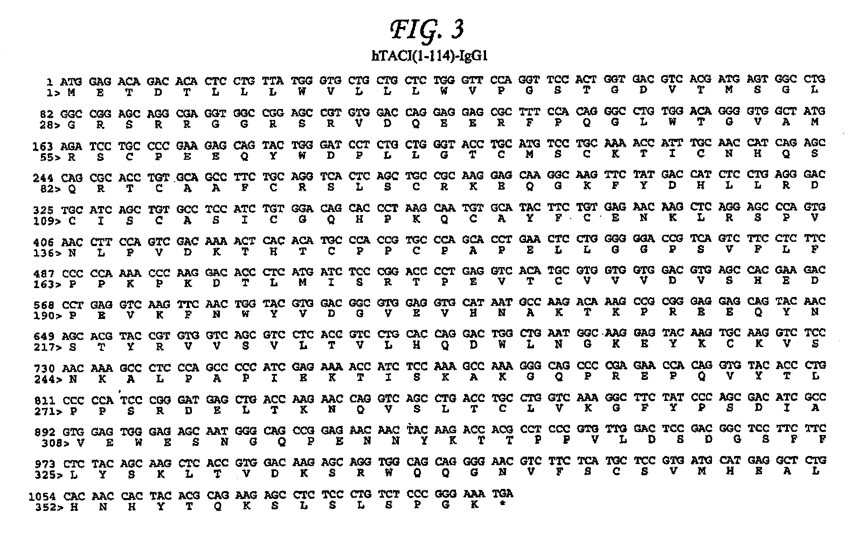

**Figure 3** is a schematic representation of the DNA sequence (SEQ ID NO:5) and its derived amino acid sequence (SEQ ID NO:6) of the TACI extracellular domain with a truncated stalk region fused to human IgG Fc. This was assembled as plasmid pJST572. The signal sequence from murine IgG-kappa, nucleotides 1-69 (amino acids 1-23), was fused in frame with the human TACI extracellular domain (amino acids 1-114 of SEQ ID NO:1) as nucleotides 70-411 (amino acids 24-137) which was fused in frame to the hlgG1 Fc as nucleotides 412-1098 (amino acids 138-366). The predicted signal peptidase cleavage site is after amino acid 20.

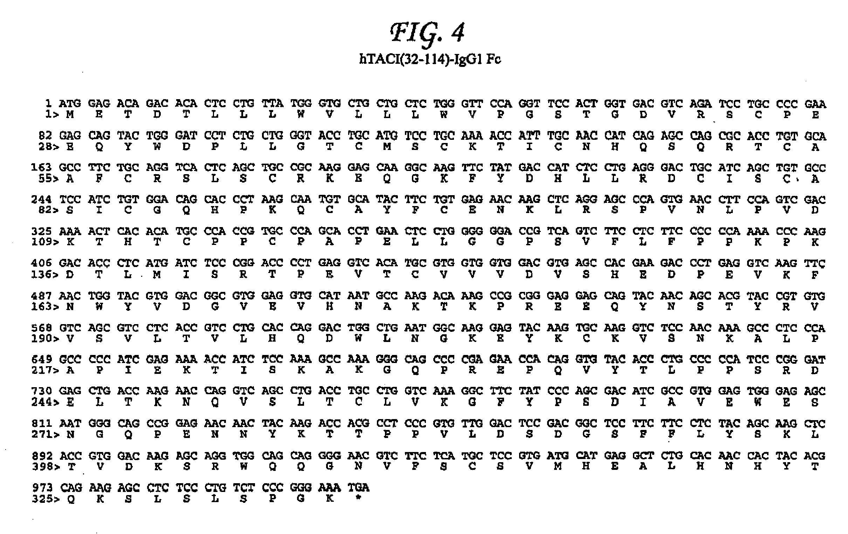

**Figure 4** is a schematic representation of the DNA sequence (SEQ ID NO:7) and its derived amino acid sequence (SEQ ID NO:8) of the TACI extracellular domain with a truncated stalk region initiating after the second methionine fused to human IgG Fc. This was assembled as plasmid pJST591. The signal sequence from murine IgG-kappa, nucleotides 1-66 (amino acids 1-22), was fused in frame with human TACI extracellular domain (amino acids 32-114 of SEQ ID NO:1) as nucleotides 67-315 (amino acids 23-105) which was fused in frame to the hIgGl Fc as nucleotides 316-1002 (amino acids 106-334). The predicted signal peptidase cleavage site is after amino acid 20.

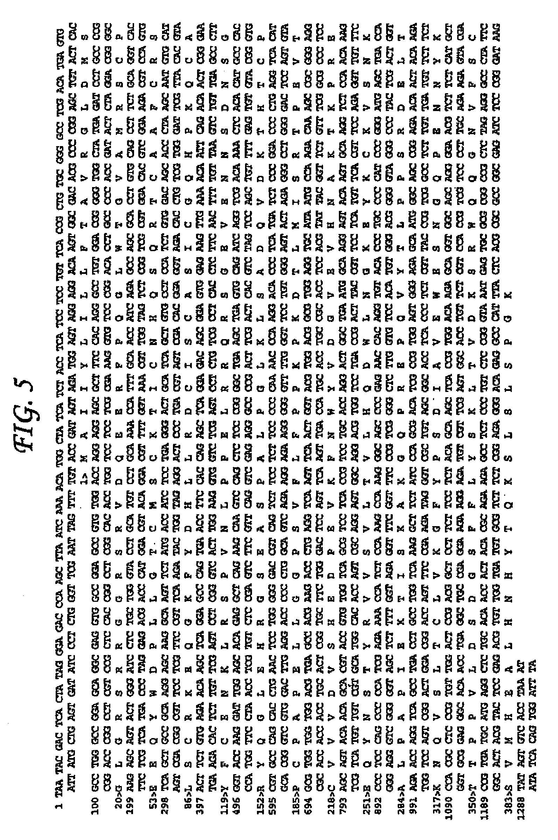

**Figure 5** is a schematic representation of the nucleic acid sequence and its derived amino acid sequence of the complete extracellular domain of TACI fused to a human IgG-Fc sequence, as assembled in plasmid PS882, wherein there is a short hemaglutinin (HA)-signal sequence in frame with the native methionine (amino acid 18) and TACI extracellular domain sequence through amino acid 177 (valine, which is amino acid 160 of TACI) after which there is a human IgG-Fc construct in frame.

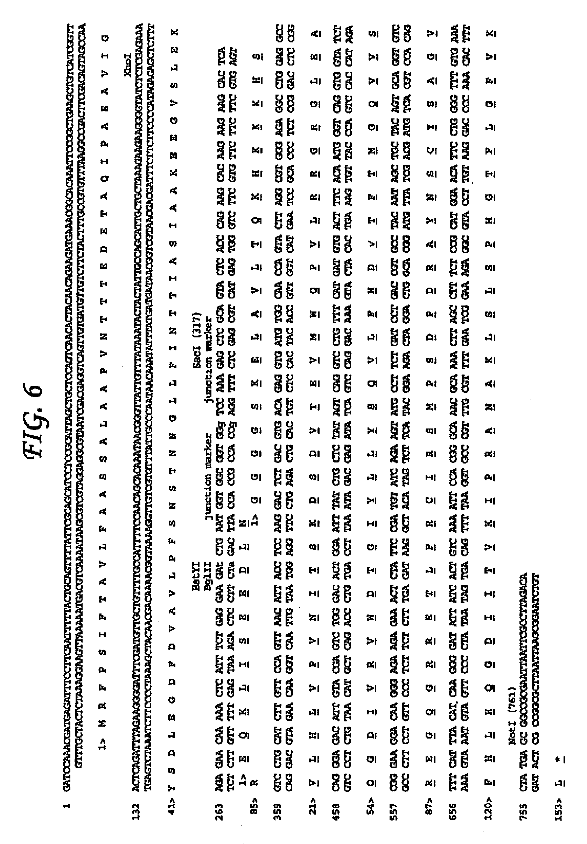

**Figure 6** is a schematic representation of the nucleic acid sequence (SEQ ID NO:4) and its derived amino acid sequence (SEQ ID NO:3) of a myc-tagged murine APRIL construct for expression in *Pichia pastoris* cells, as mapped in plasmid pCMM276, including the alpha mating factor signal sequence, which is cleaved off; the myc epitope (first 11 amino acids after the signal sequence; underlined); a short linker region (next 8 amino acids); and the soluble extracellular domain of murine APRIL coding sequence from amino acid 20, which is an alanine, to the first stop codon.

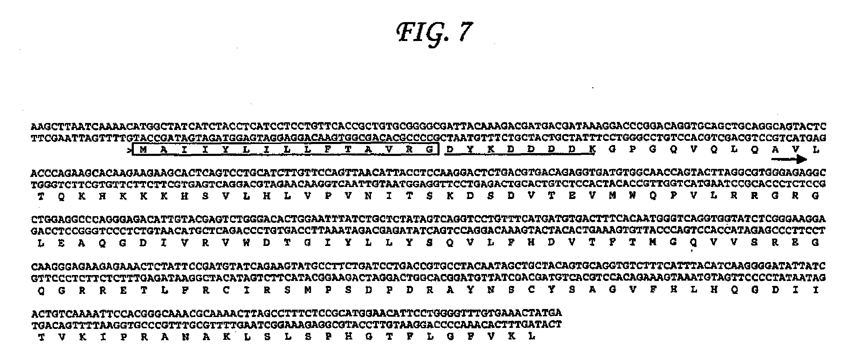

**Figure 7** is a schematic representation of the nucleic acid sequence of FLAG-tagged soluble extracellular domain of murine APRIL, and the corresponding amino acid sequence, as mapped in the mammalian expression plasmid PS784, also known as LT032, wherein there is an HA-signal sequence (boxed), the FLAG epitope (underlined), a short linker sequence, then soluble murine APRIL sequence (arrow beginning at alanine).

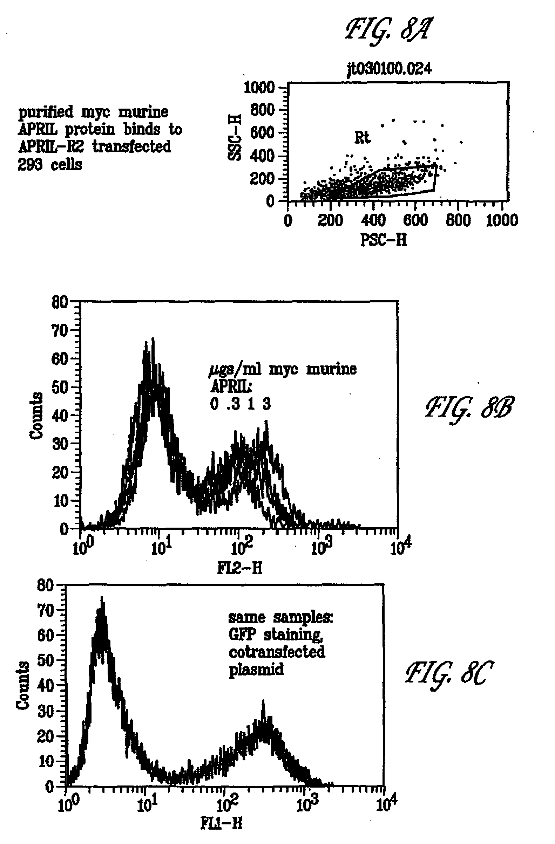

**Figure 8** is a schematic representation of purified myc-tagged murine APRIL binding to TACI transfected cells. 293EBNA cells were transfected with expression plasmid pJST552 that expressed FLAG-tagged full length human TACI. Cells were harvested 48 hours later using 5 mM EDTA and stained with myc-tagged murine APRIL at various concentrations. No protein control was stained with detection reagents (rabbit anti-murine APRIL and donkey anti-rabbit-PE (Jackson Immunoresearch)). Staining in FL1 of protein encoded by the cotransfected GFP expression plasmid illustrates expression efficiency.

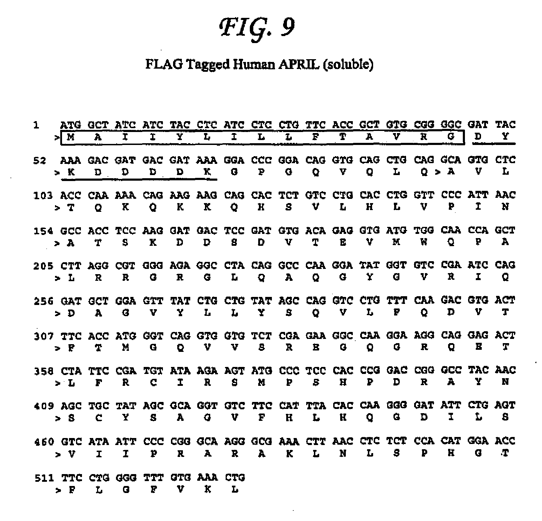

**Figure 9** is a schematic representation of the DNA sequence of FLAG tagged soluble extracellular domain of human APRIL (SEQ ID NO:9) and the corresponding amino acid sequence (SEQ ID NO:10) as cloned in the mammalian expression vector LT033. This construct contains an HA signal sequence tag (boxed), the FLAG epitope tag (underlined), and a short linker sequence fused to soluble human APRIL (arrow, beginning at alanine).

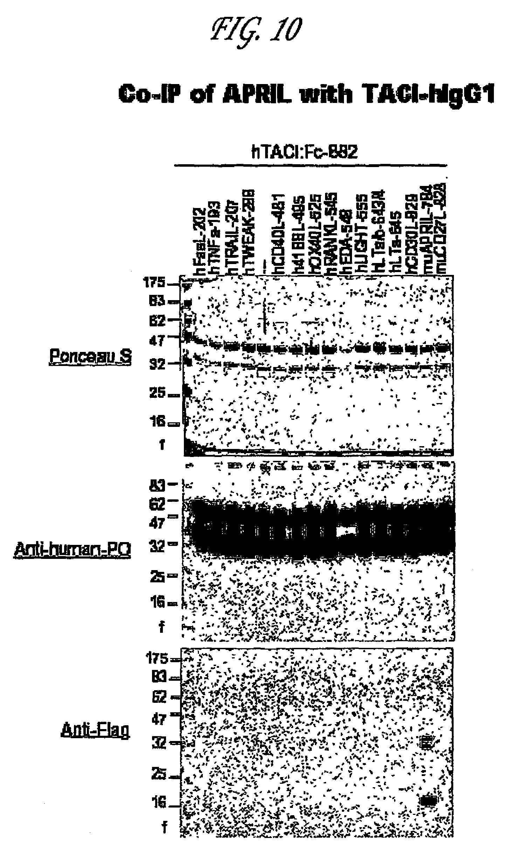

**Figure 10** is a series of representations of Western blots delineating the immunoprecipitation of FLAG-tagged murine APRIL using human TACI(1-160)-Ig (hTACI(1-160)-Ig) fusion protein. Figure 10A is a representation of a Western blot showing Ponceau-S staining of protein loads for the ligands. Figure 10B is a representation of a Western blot showing the amount of hTACI(1-160)-Ig used in the immunogrecipitations by revealing the IgG-Fc portion. Figure 10C is a representation of a Western blot showing that only APRIL immunoprecipitated with hTACI(1-160)-Ig, as evidenced by revealing the FLAG-tag.

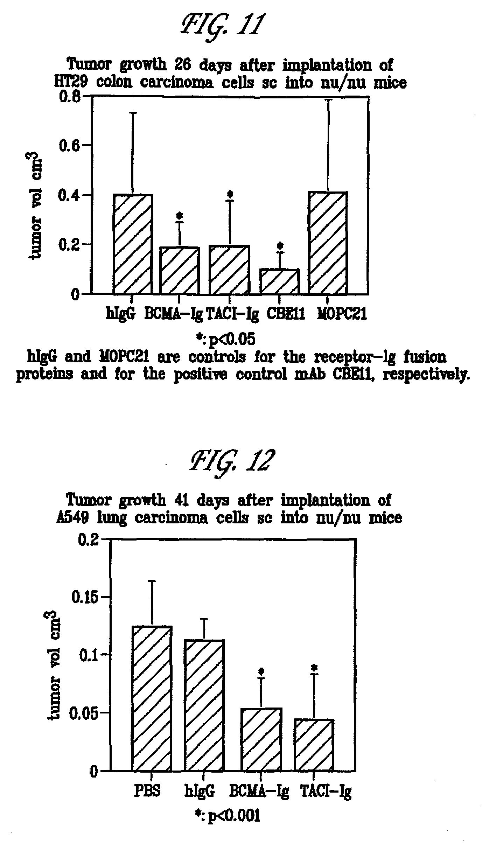

**Figure 11** shows the results of *in vivo* tumor growth inhibition of HT29 colon adenocarcinoma cells by hTACI(1-114)-Ig.

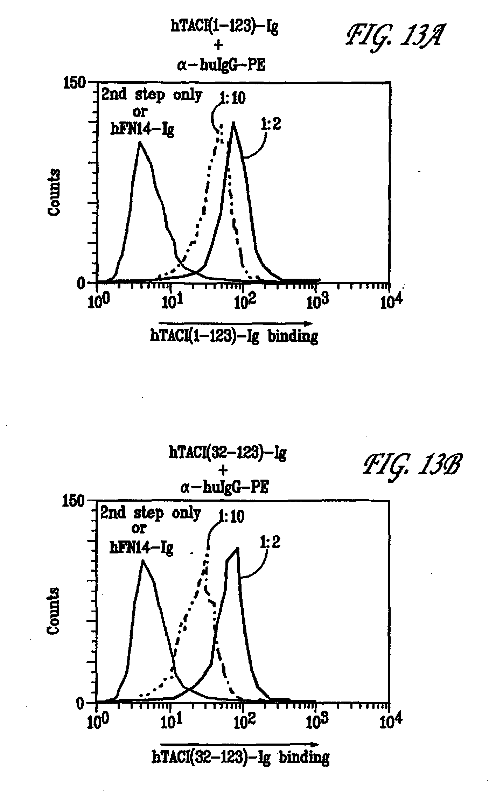

**Figure 12** shows the results of *in vivo* tumor growth inhibition of A594 lung carcinoma cells by hTACI(1-114)-Ig.

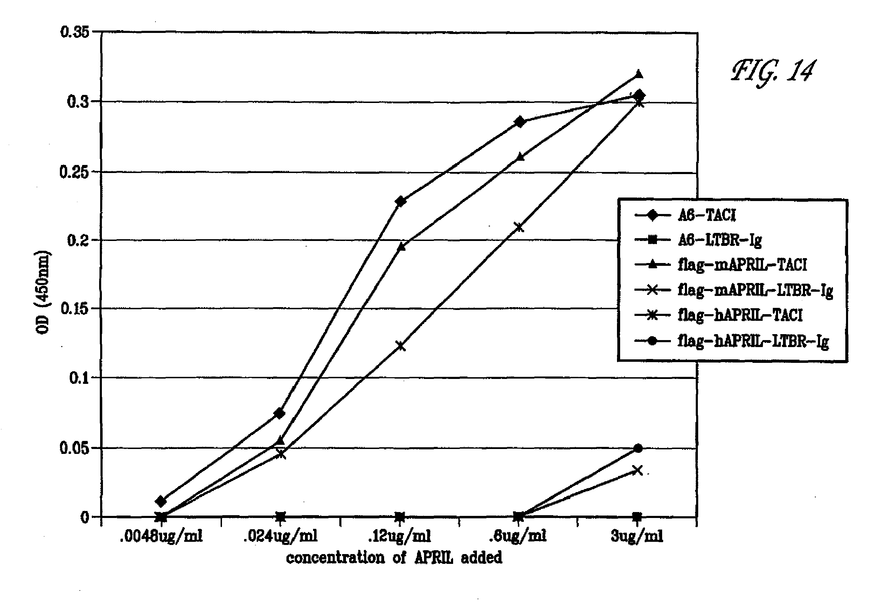

**Figure 13** shows a histogram overlay of hTACI(1-114)-Ig and hTACI(32-114)-Ig binding to cells stably expressing surface murine APRIL.
**Figure 14** shows an plot of an ELISA analysis of showing binding of murine and human APRIL to hTACI(32-114)-Ig.

### DETAILED DESCRIPTION OF THE INVENTION

The patent applications, patents and literature references cited herein indicate the knowledge of those of ordinary skill in this field and are hereby incorporated by reference in their entirety. In the case of inconsistencies between any reference cited herein and the specific teachings of the present disclosure, this disclosure will prevail. Similarly, any inconsistencies between an art-understood meaning of a term and a meaning of a term as specifically taught in the present disclosure will be resolved in favor of this disclosure.

It has now been unexpectedly discovered that TACI is a receptor for APRIL (A Proliferation Inducing Ligand). It is also an unexpected discovery of the present invention that TACI reagents can be used to treat a mammal for a condition associated with undesired cell proliferation comprising administering to said mammal an effective amount of a TACI reagent, wherein said reagent extends mean survival time of said mammal by about 10 %, 15%, 20%, 25% or more compared to the absence of administering the TACI reagent for said condition. Moreover, it is an unexpected discovery of the present invention that TACI reagents can be used to reduce the size of a tumor located on or in a mammal comprising administering to said mammal an effective amount of a TACI reagent, wherein said reagent reduces the size of said tumor by about 10 %, 15%, 20%, 25% or more as compared to not administering the TACI reagent.

As used herein, "treating or treatment" means an approach for obtaining beneficial or desired clinical results. For purposes of this invention, beneficial or desired clinical results include, but are not limited to, one or more of the following: alleviation of symptoms, diminishment of extent of disease, stabilized (*e.g.,* not worsening) state of disease, preventing spread (*e.g.*, metastasis) of disease, preventing occurrence or recurrence of disease, delay or slowing of disease progression, amelioration of the disease state, and remission (whether partial or total). Also encompassed by "treatment" is a reduction of pathological consequences of a condition associated with undesired cell proliferation, including specifically, cancer.

By "mammal" as used herein means any mammal including humans, cows, horses, dogs, rats, mice and cats. In preferred embodiment of the invention, the mammal is a human.

"A condition associated with undesired cell proliferation" as used herein includes but is not limited to cancer, specifically, conditions comprising at least one solid tumor, including, but not limited to renal cell cancer, Kaposi's sarcoma, breast cancer, sarcoma, ovarian carcinoma, rectal cancer, throat cancer, melanoma, colon cancer, bladder cancer, mastocytoma, lung cancer, mammary adenocarcinoma, pharyngeal squamous cell carcinoma, gastrointestinal cancer or stomach cancer. Preferably, the cancer is mastocytoma, melanoma, lymphoma, mammary adenocarcinoma, prostate and breast cancer. Also contemplated are other conditions associated with undesired cell proliferation including but not limited to cellular hyperproliferation (hyperplasia), selected from the group consisting of, for example, scleroderma, pannus formation in rheumatoid arthritis, post-surgical scarring and lung, liver, and uterine fibrosis.

As used herein "administering" means the TACI reagent can be administered alone or in combination with other pharmaceutical agents and can be combined with a physiologically acceptable carrier therefor. The effective amount and method of administration of the particular TACI reagent can vary based on the individual mammal and the stage of the disease and other factors evident to one skilled in the art. The route(s) of administration useful in a particular application are apparent to one of skill in the art.

Routes of administration include but are not limited to topical, transdermal, parenteral, gastrointestinal, transbronchial and transalveolar. Topical administration is accomplished via a topically applied cream, gel, rinse, etc. containing an oligonucleotide conjugate. Transdermal administration is accomplished by application of a cream, rinse, gel, *etc.* capable of allowing the TACI reagent to penetrate the skin and enter the blood stream. Parenteral routes of administration include but are not limited to electrical or direct injection such as direct injection into a central venous line, intravenous, intramuscular, intraperitoneal or subcutaneous injection. Gastrointestinal routes of administration include but are not limited to ingestion and rectal administration. Transbronchial and transalveolar routes of administration include but are not limited to inhalation, either via the mouth or intranasally.

An "effective amount" as used herein is an amount sufficient to effect beneficial or desired clinical results (Stites *et al.,* BASIC & CLINICAL IMMUNOLOGY, Lange Medical Publications, Los Altos, California, 1982). An effective amount can be administered in one or more administrations as described herein. For purposes of this invention, an effective amount of a TACI reagent is an amount sufficient to extend mean survival time of a mammal by at least 10%, alternatively 15%, 20% or 25% in comparison to mean survival in the absence of administering a TACI reagent. Detection and measurement of indicators of efficacy are generally based on measurement of clinical symptoms associated with the disease state, such as increased average life expectancy after treatment with a TACI reagent.

An effective amount of a TACI reagent for reducing the size of a tumor in or on a mammal is an amount sufficient to reduce tumor size on or in a mammal by at least 10%, alternatively 15%, 20% or 25% more than in the absence of administering a TACI reagent. Methods for measuring tumor size in a mammal are known to those of skill in the art and can be measured by non-invasive procedures, including but not limited to using a micrometer to measure the tumor diameter, if the tumor is located on the exterior surface of a mammal. Alternatively if the tumor is located in the interior of the mammal one can use MRI to measure the tumor diameter. Invasive procedures include surgically removing the tumor from the mammal and weighing the tumor and comparing the size of the tumor to pretreatment with the TACI reagent.

As used herein "a TACI reagent" means those reagents that can influence how the TACI signal is interpreted within the cell including antagonistic TACI reagents that can diminish ligand binding to TACI, including for example, TACI fusion proteins such as TACI-IgG Fc. Also contemplated are agonistic TACI reagents that can augment ligand binding to TACI, including for example, antibodies to TACI such as anti-TACI monoclonal antibodies. The term Fc domain refers to a part of the molecule comprising the hinge, CH2 and CH3 domains, but lacking the antigen binding sites. The term is meant to include the equivalent regions of an IgG, an IgM and other antibody isotypes.

Another aspect of the invention relates to the use of the polypeptide encoded by the isolated nucleic acid encoding TACI in "antisense" therapy. As used herein, "antisense" therapy refers to administration or *in situ* generation of oligonucleotides or their derivatives which specifically hybridize under cellular conditions with the cellular mRNA and/or DNA encoding the ligand of interest, so as to inhibit expression of the encoded protein, *i.e.,* by inhibiting transcription and/or translation. The binding may be by conventional base pair complementarity, or, for example, in the case of binding to DNA duplexes, through specific interactions in the major groove of the double helix. In general, "antisense" therapy refers to a range of techniques generally employed in the art, and includes any therapy that relies on specific binding to oligonucleotide sequences.

An antisense construct of the present invention can be delivered, for example, as an expression plasmid, which, when transcribed in the cell, produces RNA that is complementary to at least a portion of the cellular mRNA which encodes TACI. Alternatively, the antisense construct can be an oligonucleotide probe that is generated *ex vivo.* Such oligonucleotide probes are preferably modified oligonucleotides that are resistant to endogenous nucleases, and are, therefore, stable *in vivo.* Exemplary nucleic acids molecules for use as antisense oligonucleotides are phosphoramidates, phosphothioate and methylphosphonate analogs of DNA (See, e.g.*,* U.S. Patent Nos. 5,176,996; 5,264,564; and 5,256,775). Additionally, general approaches to constructing oligomers useful in antisense therapy have been reviewed, for example, by Van Der Krol et al., (1988) Biotechniques 6:958-976; and Stein et al. (1988) Cancer Res. 48:2659-2668, specifically incorporated herein by reference. In some embodiments the antisense oligonucleotides are complementary to a regulatory region of the mRNA that encodes TACI. In other embodiments, the antisense oligonucleotides are complementary to a protein encoding portion of the mRNA encoding TACI. In some embodiments of the invention the antisense oligonucleotides are about 12 to about 35 nucleotides in length. In other embodiments, the antisense oligonucleotides are about 15 to about 25 nucleotides in length. In other embodiments, the antisense oligonucleotides are about 17 to about 22 nucleotides in length.

As used herein, "extend mean survival time" means that the average life expectancy associated with a particular condition associated with undesired cell proliferation is on average increased. Average life expectancy is known to those of skill in the art for various forms of cancer in various forms of mammals, including various forms of cancer in humans, and various forms of cancer in rodents, including mice. Furthermore, as used herein, an extended mean survival time of, for example, about 10% or more as compared to mean survival time in the absence of administering a TACI reagent, means for example, that for a human patient with a form of cancer that has an survival time of about 365 days (1 year) in the absence of treatment, a TACI reagent would increase their average life expectancy by about 10% of 365 days or more, for a total of about 400 days total survival.

By "soluble TACI reagent" means a soluble form of a TACI protein or polypeptide fragment in which the transmembrane domain has been cleaved or mutated by standard biochemical or recombinant DNA techniques such that it is soluble.

In another aspect, the invention provides a method of treating a mammal for a condition associated with undesired cell proliferation comprising administering to the mammal an effective amount of a substantially pure, soluble form of a TACI protein or polypeptide fragment of a TACI protein, wherein the TACI protein or polypeptide fragment of the TACI protein binds the extracellular domain of APRIL and thereby inhibits aberrant cell growth. By "protein" or "polypeptide" means any molecule comprising two or more amino acids joined together with a peptide bond, regardless of length or post-translational modifications (*e.g.*, glycosylation, lipidation, acetylation, or phosphorylation).

As used herein, by "polypeptide fragment" means a polypeptide that is shorter in length than the full length protein from which it was derived but greater than a single amino acid. Hence, a polypeptide fragment of a TACI protein has less amino acids than the full length TACI protein. For example "hTACI(1-160)" refers to a human TACI polypeptide sequence containing amino acid residues 1 through 160 of human TACI. "hTACI(1-114)" refers to a human TACI polypeptide sequence containing amino acid residues 1 through 123 of human TACI. "hTACI(32-114)" refers to a human TACI polypeptide sequence containing amino acid residues 32 through 123 of human TACI. In embodiments in which a TACI fragment is joined as a fusion protein to a portion of an immunoglobulin molecule, "-Ig" is added to the end of the designation for the TACI fragment (*e.g.,* "hTACI(1-160)-Ig," "hTACKI(1-114)-Ig," and "hTACI(32-114)-Ig") to indicate the fusion protein.

A preferred polypeptide fragment of a TACI protein is the soluble extracellular domain of TACI. For example, a preferred polypeptide fragment of a TACI protein is, without limitation, amino acids 1 to about 166 (*e.g.* 1 to about 161 or 1 to about 171) and other amino acids in between. Other preferred polypeptide fragments include, but are not limited to amino acids 1 to about 114 and amino acids from about 32 to about 114. A preferred polypeptide fragment of TACI is one that binds to the extracellular domain of APRIL.

The claimed invention in certain embodiments includes methods of using peptides derived from TACI which have the ability to bind to APRIL. Fragments of TACI can be produced in several ways, *e.g.*, recombinantly, by PCR, proteolytic digestion or by chemical synthesis. Internal or terminal fragments of a polypeptide can be generated by removing one or more nucleotides from one end or both ends of a nucleic acid that encodes the polypeptide. Expression of the mutagenized DNA produces polypeptide fragments.

Polypeptide fragments can also be chemically synthesized using techniques known in the art such as conventional Merrifield solid phase f-moc or t-boc chemistry. For example, peptides and DNA sequences of the present invention may be arbitrarily divided into fragments of desired length with no overlap of the fragment, or divided into overlapping fragments of a desired length. Methods such as these are described in more detail below.

Soluble forms of the TACI can often signal effectively and, hence, can be administered as a drug which now mimics the natural membrane form. It is possible that the TACI claimed herein are naturally secreted as soluble cytokines, however, if not, one can reengineer the gene to force secretion. To create a soluble secreted form of TACI, one would remove at the DNA level the N-terminus transmembrane regions, and some portion of the stalk region, and replace them with a type I leader or alternatively a type II leader sequence that will allow efficient proteolytic cleavage in the chosen expression system. A skilled artisan could vary the amount of the stalk region retained in the secretion expression construct to optimize both ligand binding properties and secretion efficiency. For example, the constructs containing all possible stalk lengths, *i.e.,* N-terminal truncations, could be prepared. In certain embodiments, proteins starting at amino acids 1 to 32 are produced. The optimal length stalk sequence would result from this type of analysis.

By "substantially pure" or "substantially purified" is meant a compound (*e.g.*, a nucleic acid molecule or a protein) that has been separated from components (*e.g.*, nucleic acid molecules, proteins, lipids, and/or carbohydrates) which naturally accompany it. Water, buffers, and other small molecules (*e.g.,* molecules having a molecular weight of less than about 1000 daltons) may accompany a substantially pure compound of the invention. Preferably, a substantially purified compound is at least 70%, by weight, free from components which naturally accompany it. More preferably, a substantially purified compound is at least 75%, by weight, free from components which naturally accompany it; still more preferably, at least 80%, by weight, free; even more preferably, at least 85%, by weight, free; and even more preferably, at least 90%, by weight, free from components which naturally accompany it. Most preferably, a substantially purified compound is at least 95%, by weight, free from components which naturally accompany it. In certain embodiments of the second aspect of the invention, the polypeptide fragment of the TACI protein has an amino acid sequence that is included within the extracellular domain of TACI. This fragment may be any size that is smaller than the TACI extracellular domain. Thus, this fragment may include from about 26% to about 99% of the extracellular domain, and so may include any part of amino acids 1 to about 166 by SEQ ID NO:1. For example, a polypeptide fragment of the invention includes the N-terminal amino acid residues 1 to about 166 of SEQ ID NO:1. In other embodiments, the polypeptide fragment of the invention includes the N-terminal amino acid residues 1 to about 114 of SEQ ID NO:1. In other embodiments, the polypeptide fragment of the invention includes the N-terminal amino acid residues from about 32 to about 114 of SEQ ID NO:1.

The "TACI extracellular domain" refers to a form of a TACI protein or polypeptide which is essentially free of transmembrane and cytoplasmic domains of TACI. Ordinarily, TACI extracellular domain have less than 1% of such transmembrane and cytoplasmic domains and preferably have less than 0.5% of such domains. In a preferred embodiment, the TACI extracellular domain is amino acids 1 to about 166 of SEQ ID NO:1. It is understood by the skilled artisan that the transmembrane domain identified for the TACI protein or polypeptide fragment of the present invention is identified pursuant to criteria routinely employed in the art for identifying that type of hydrophobic domain. The exact boundaries of a transmembrane domain vary but most likely by no more than about five amino acids at either end of the domain specifically mentioned herein.

In accordance with this second aspect, the invention provides all derivative, mutants, truncations, and/or splice variants of TACI, so long as these derivatives, mutants, truncations, and/or splice variants share at least 26% amino acid sequence identity with SEQ ID NQ:1, preferably, at least 30% sequence identity, more preferably at least 50% sequence identity, more preferably at least 65% sequence identity, more preferably, at least 70% sequence identity, more preferably, at least 75% sequence identity, still more preferably, at least 80% sequence identity, and even more preferably at least 85% sequence identity, and still even more preferably, at least 90% sequence identity, and most preferably, at least 95% sequence identity with SEQ ID NO:1, using the sequence of the TACI derivative, mutant, truncation, and/or splice variants as the probe. "Sequence identity" with respect TACI amino acid sequences identified herein is defined as the percentage of amino acid residues in a candidate sequence that are identical with the amino acid residues in the TACI amino acid sequence, after aligning the sequences and introducing gaps, if necessary, to achieve the maximum percent sequence identity, and not considering any conservative substitutions as part of the sequence identity. Alignment for purposes of determining percent amino acid sequence identity is achieved in various ways that are within the skill in the art, for instance, using publicly available computer software such as BLAST, ALIGN, or Megalign (DNASTAR) software. Those skilled in the art determine appropriate parameters for measuring local alignment, including any algorithms needed to achieve maximum alignment over the full length of the sequences being compared, to detect relationships among sequences which share only isolated regions of similarity. (Altschul et al. (1990) J. Mol. Biol. 215:403-410).

Accordingly, derivative, mutants, truncations, and/or splice variants of a TACI protein displaying substantially equivalent or altered activity are likewise contemplated. These variants may be deliberate, for example, such as modifications obtained through site-directed mutagenesis, or may be accidental, such as those obtained through mutations in hosts that are producers of the protein. Included within the scope of these terms are proteins specifically recited herein, as well as all substantially homologous analogs and allelic variations.

In one non-limiting example, in accordance with the invention, a soluble, substantially pure TACI protein consisting essentially of amino acid residues 1 to about 166 of SEQ ID NO:1, or soluble variations thereof, is chemically synthesized according to standard techniques (*e.g.,* at a commercial peptide generating facility). Alternatively, a soluble, substantially pure TACI protein or polypeptide fragment thereof, is synthesized by standard, well-known recombinant DNA techniques in prokaryotic or eucaryotic host cells.

Analogs of TACI can differ from the naturally occurring TACI in amino acid sequence, or in ways that do not involve sequence, or both. Non-sequence modifications include *in vivo* or *in vitro* chemical derivatization of TACI. Non-sequence modifications include, but are not limited to, changes in acetylation, methylation, phosphorylation, carboxylation or glycosylation.

Preferred analogs include TACI, biologically active fragments thereof, whose sequences differ from the sequence given in SEQ ID NO:1, by one or more conservative amino acid substitutions, or by one or more non-conservative amino acid substitutions, deletions or insertions which do not abolish the activity of TACI. Conservative substitutions typically include the substitution of one amino acid for another with similar characteristics (*e.g.*, substitutions within the following groups: valine, glycine; glycine, alanine; valine, isoleucine, leucine; aspartic acid, glutamic acid; asparagine, glutamine; serine, threonine; lysine, arginine; and, phenylalanine, tyrosine.

In another aspect, the invention encompasses a method of treating cancer in a mammal comprising administering an effective amount of a soluble, substantially pure fusion protein comprising a soluble form of a TACI protein or polypeptide fragment thereof, wherein the fusion protein inhibits cell growth. In certain embodiments, the TACI fusion protein has an amino acid sequence comprising the extracellular domain of SEQ ID NO:1, or a portion of the extracellular domain.

By "fusion protein" means a protein that comprises at least two segments of a protein or polypeptide fragment joined together by any means, including, without limitation, a covalent bond (*e.g.,* peptide bond), a non-covalent bond (*e.g.,* ionic bond or hydrogen bond) or by a chemical crosslinker. Also, any variety of fusion proteins carrying only the extracellular domain of the TACI protein can be generated. Non-limiting examples include a fusion protein comprising the extracellular domain of the TACI protein and an immunoglobulin polypeptide, including for example, the immunoglobulin polypeptide IgG.

The invention also includes antibodies specifically reactive with the claimed TACI reagents. Anti-protein/anti-peptide antisera or monoclonal antibodies can be made by standard protocols (See, for example, ANTIBODIES: A LABORATORY MANUAL Harlow and Lane, Eds., Cold Spring Harbor Press, N.Y., 1988). A mammal such as a mouse, a hamster or rabbit can be immunized with an immunogenic form of the peptide. Techniques for conferring immunogenicity on a protein or peptide include conjugation to carriers, or other techniques, well known in the art.

An immunogenic portion of TACI can be administered in the presence of an adjuvant. The progress of immunization can be monitored by detection of antibody titers in plasma or serum. Standard ELISA or other immunoassays can be used with the immunogen as antigen to assess the levels of antibodies.

In a preferred embodiment, the subject antibodies are immunospecific for antigenic determinants of TACI, *e.g.,* antigenic determinants of a polypeptide of SEQ ID NO:1, or a closely related human or non-human mammalian homolog (*e.g.,* 70, 80 or 90 percent homologous, more preferably at least 95 percent homologous). In yet a further preferred embodiment of the present invention, the anti-TACI antibodies do not substantially cross react (i.e., react specifically) with a protein which is for example, less than 80 percent homologous to SEQ ID NO:1; preferably less than 90 percent homologous with SEQ ID NO:1; and, most preferably less than 95 percent homologous with SEQ ID NO:1. By "not substantially cross react," it is meant that the antibody has a binding affinity for a non-homologous protein which is less than 10 percent, more preferably less than 5 percent, and even more preferably less than 1 percent, of the binding affinity for a protein of SEQ ID NO.1.

The term antibody as used herein is intended to include fragments of antibodies which are also specifically reactive with TACI. Antibodies can be fragmented using conventional techniques and the fragments screened for utility in the same manner as described above for whole antibodies. For example, F(ab')₂ fragments can be generated by treating antibody with pepsin. The resulting F(ab')₂ fragment can be treated to reduce disulfide bridges to produce Fab' fragments. The antibodies of the present invention are further intended to include biospecific and chimeric molecules having anti-TACI activity. Thus, both monoclonal and polyclonal antibodies (Ab) directed against TACI, and antibody fragments such as Fab' and F(ab')₂, can be used to block the action of the TACI.

Various forms of antibodies can also be made using standard recombinant DNA techniques. (Winter and Milstein, (1991) Nature 349:293-299, specifically incorporated by reference herein). For example, chimeric antibodies can be constructed in which the antigen binding domain from an animal antibody is linked to a human constant domain (*e.g.*, U.S. Patent No. 4,816,567, to Cabilly et al.*,* incorporated herein by reference). Chimeric antibodies may reduce the observed immunogenic responses elicited by animal antibodies when used in human clinical treatments.

In addition, recombinant "humanized antibodies" which recognize TACI can be synthesized. Humanized antibodies are chimeras comprising mostly human IgG sequences into which the regions responsible for specific antigen-binding have been inserted. Animals are immunized with the desired antigen, the corresponding antibodies are isolated, and the portion of the variable region sequences responsible for specific antigen binding are removed. The animal-derived antigen binding regions are then cloned into the appropriate position of human antibody genes in which the antigen binding regions have been deleted. Humanized antibodies minimize the use of heterologous (*i.e.,* inter species) sequences in human antibodies, and thus are less likely to elicit immune responses in the treated subject.

Construction of different classes of recombinant antibodies can also be accomplished by making chimeric or humanized antibodies comprising variable domains and human constant domains (CH1, CH2, CH3) isolated from different classes of immunoglobulins. For example, antibodies with increased antigen binding site valencies can be recombinantly produced by cloning the antigen binding site into vectors carrying the human heavy chain constant regions. (Arulanandam et al., (1993) J. Exp. Med. 177:1439-1450, incorporated herein by reference).

In addition, standard recombinant DNA techniques can be used to alter the binding affinities of recombinant antibodies with their antigens by altering amino acid residues in the vicinity of the antigen binding sites. The antigen binding affinity of a humanized antibody can be increased by mutagenesis based on molecular modeling. (Queen et al., (1989) Proc. Natl. Acad. Sci. USA 86:10029-10033) incorporated herein by reference.

The present invention also provides pharmaceutical compositions comprising a TACI polypeptide and a pharmaceutically acceptable excipient. Suitable carriers for a TACI polypeptide, for instance, and their formulations, are described in REMINGTON' PHARMACEUTICAL SCIENCES, 16th ed., Oslo et al. Eds., Mack Publishing Co., 1980. Typically, an appropriate amount of a pharmaceutically acceptable salt is used in the formulation to render the formulation isotonic. Examples of the carrier include buffers such as saline, Ringer's solution and dextrose solution. The pH of the solution is preferably from about 5 to about 8, and more preferably from about 7.4 to about 7.8. Further carriers include sustained release preparations such as semipermeable matrices of solid hydrophobic polymers, which matrices are in the form of shaped articles, *e.g.,* liposomes, films, or microparticles. It will be apparent to those of skill in the art that certain carriers may be more preferable depending upon for instance the route of administration and concentration of the TACI polypeptide being administered.

Administration may be accomplished by injection (*e,g.,* intravenous, intraperitoneal, subcutaneous, intramuscular) or by other methods such as infusion that ensure delivery to the bloodstream in an effective form.

In another aspect of the invention, the invention is directed to a method of treating a mammal for a condition associated with undesired cell proliferation, comprising administering to the mammal an effective amount of a substantially pure binding agent that specifically binds a APRIL protein, wherein the binding of the binding agent to APRIL inhibits undesired cell proliferation. The binding agent in this aspect is a protein having at least 26% sequence identity with amino acid residues 1 to about 166 of SEQ ID NO:1. Preferably, the binding agent shares at least 30% sequence identity, more preferably at least 50% sequence identity, more preferably at least 65% sequence identity, more preferably, at least 70% sequence identity, more preferably, at least 75% sequence identity, still more preferably, at least 80% sequence identity, and even more preferably at least 85% sequence identity, and still even more preferably, at least 90% sequence identity, and most preferably, at least 95% sequence identity with SEQ ID NO:1, using the sequence of the TACI derivative, mutant, truncation, and/or splice variant as the probe. In certain embodiments, the APRIL protein has an amino acid sequence described in WO 99/12965. In certain preferred embodiments, the binding agent is an antibody, such as a polyclonal antibody, or a monoclonal antibody, or a recombinant, humanized, or chimeric antibody, or a fragment of an antibody that specifically binds an APRIL protein or a extracellular domain thereof.

The TACI reagents are administered in an effective amount which may easily be extrapolated by the animal data provided herein by methods known to those of ordinary skill in the art (*e.g.,* based on body weight, body surface area). Furthermore, it is in the purview of the skilled physician to increase or decrease amounts of the TACI reagent to achieve the desired effects without causing any undesirable side effects.

The following Examples are provided to illustrate the present invention, and should not be construed as limiting thereof.

### EXAMPLES

The following methods and materials were used in the Examples disclosed hereinafter.

### I. Methods and Materials

### A. Cloning and expression of myc-tagged A88 murine APRIL in Pichia pastoris

The expression vector pCCM276 (Figure 4), was constructed by polymerase chain reaction using pCCM213.10 (Myc-tagged-H98 muAPRIL) as template and synthetic oligonucleotides CDL620 and LTB619 as forward and reverse primers, respectively. Forward primer CDL620 adds back 10 amino acids of murine APRIL sequence to represent the native cleaved form, along with a FAS ligand derived KEL motif and Sac1 site. Resultant amplified fragments were digested with *Sac*I and *Not*I, gel purified and ligated into those same restriction sites of pCCM213.10. This expression construct contains yeast signal sequence alpha mating factor directly fused to myc epitope tag, KEL motif and murine APRIL starting at Alanine 88. pCCM2786 was linearized with *Stu*I, electroporated into GS115 strain (his4-) and plated onto minimal media containing dextrose. HIS4 transformants were analyzed for protein expression by inoculating a single representative colony in rich media (BMGY) and allowing it to grow to density for 48 hours at 30°C. Cultures were spun, and cell pellets were resuspended (1:5) in a rich induction media containing 2% methanol (BMMY). After two days of induction at 30°C, supemates were run out on SDS-PAGE and assessed for the presence of muAPRIL. Coomassie blue staining and Western Blot (with the anti-myc mAB 9E10) analysis confirmed the presence of the glycosylated A88 myc-tagged murine APRIL.

### B. Myc-Murine APRIL A88 Purification

Myc-tagged murine April (myc-muAPRIL) was expressed in *P. pastoris.* The protein has an isoelectric point of about 7.45. 175 ml of *Pichia* supernate was dialyzed into PBS overnight with a 30kD cutoff dialysis membrane. The dialysate was then passed through a Q sepharose column. The myc-muAPRIL was collected in the column flow through, while contaminants were bound to the column. The eluted myc-muAPRIL was concentrated 20 fold and then chromatographed on a superdex 75 gel filtration column. After gel filtration, 8 mg of myc-muAPRIL were recovered having an OD of 1.0AU-1 mg of myc-muAPRIL. Coomassie stained SDS PAGE showed a homogenous preparation of myc-muAPRIL with two bands migrating at molecular weights of approximately 22 kD and 18 kD. Western blot analysis using mouse monoclonal 9E10 antibody (anti-myc) showed that both bands observed were immunoreactive. The expected N-terminus of the purified myc-muAPRIL was verified by Edman degradation of the blotted protein. The N-terminal sequencing and immunoreactivity to 9E10 prove the myc tag was present on the myc-muAPRIL N-termini.

### C. FLAG-Murine APRIL Purification

Plasmid PS784 (also called LT032) (Figure 7) was used to transiently transfect 293 T cells using lipofectamine reagent (Gibco-BRL) and serum free media. The plasmid, constructed in the mammalian expression vector PCR3 (Invitrogen) encodes the soluble receptor-binding domain of murine APRIL, with an N-terminal FLAG-tag, into the cell culture media. FLAG-APRIL protein was purified from serum free media using an anti-FLAG mAb M2 column and excess purified FLAG peptide, following the manufacturers' instructions (Kodak). Alternatively murine APRIL and other FLAG-tagged ligands were analyzed directly from conditioned media.

### D. hTACI(1-160)-Ig Expression

A plasmid encoding soluble hTACI(1-160)-Ig (PS882; Figure 5) was used to transiently to transfect 293 cells. Conditioned media from 293 cells overexpressing hTACI(1-160)-Ig was used in the immunoprecipitation analysis to detect binding to APRIL protein.

### E. Full Length TACI Expression

A plasmid encoding flag-tagged full length TACI (pJST552; Figure 2) was transiently transfected into 293 cells. The full length form of the molecule is retained on the cell surface. These transfected cells were used in FACS analyses to detect binding to APRIL protein.

### F. FLAG-human APRIL production

Plasmid LT033 (Figure 9) was used to transiently transfect 293 T cells using Lipofectamine reagent (Gibco-BRL) and serum-free media. The plasmid, constructed in mammalian expression vector PCR3 (Invitrogen) encodes the soluble receptor-binding domain of human APRIL, with an N-terminal FLAG-tag, and the expressed protein is secreted into the cell culture media. FLAG human APRIL was analyzed directly from cell culture supernate.

### EXAMPLE 1

### Human TACI Expressed on Cells Interacts With Myc-Tagged Murine APRIL

293EBNA cells were co-transfected with a plasmids expressing full length N-terminally FLAG tagged human TACI (pJST552)(see Figure 2), and a GFP marker, (AN050). Transfections were performed using Lipofectamine 2000 (LifeTechnologies) according to manufacturer's conditions. Transfection media was DMEM supplemented with 10% FBS, 4 mM glutamine, and 50 µM Z-VAD (BACHEM Bioscience Inc.) Cells were harvested with PBS supplemented with 5 mM EDTA at 24 hours post-transfection. Cells were washed in FACS buffer (PBS-10% FBS-0.02% NaN₃) and resuspended at a density of 5x10⁶ cells/mL. Expression of TACI was verified by staining 100 microliters of transfected cells with anti-FLAG mAb at 5µg/ml on ice for 30 minutes followed by donkey anti-mouse IgG-PE at 1:100 (Jackson ImmunoResearch). Cells were assayed for their ability to bind APRIL over a concentration range of 3 µg/ml to 300 ng/ml by incubating on ice in FACS buffer for 30 minutes. Binding was revealed using Rb1532, a rabbit polyclonal Ab raised to mAPRIL (1:100), followed by donkey anti-rabbit IgG-PE (1:100, Jackson ImmunoResearch). 7-AAD was included in the terminal stain and used to gate out dead cells. The samples were analyzed by FACS and plotted (Figure 8). Cell gate analyzed is shown as R1, Specific staining is seen at the three different concentrations of myc-tagged murine APRIL protein shown, as compared to no protein control (Figure 8).

### EXAMPLE 2

### Soluble hTACI(1-160)-Ig Interacts with FLAG-Tagged Murine APRIL

250 µl of Optimem containing hTACI(1-160)-Ig was mixed with various FLAG ligands of the TNF family. Some of the ligands were in Optimem while others were anti-FLAG (mAb M2)-purified. The common characteristic was that the amount used was empirically the same (500 ng for purified ligands, *i.e.* hBAFF, muBAFF, TRAIL, FasL, EDA). Ligands were produced in bacteria (hBAFF, TRAIL) or transiently expressed in 293 EBNA cells (all others). Control Receptors-Fc were mostly used as Optimem supemates, except TNFR1, OX40, LTBR, which were purified over protein-A columns. 1 µg of purified receptors was used. Receptor-Fc proteins (about 1 µg) were mixed with FLAG-ligands (about 500 ng). Volume was adjusted to 1.2 ml with PBS and 5 µl of protein A-Sepharose was added. Incubation was performed for 1 h at 4°C on a wheel. Beads were harvested by centrifugation and loaded onto a minicolumn (yellow tips with 1 mm diameter frit). Washes were performed by applying vacuum at the bottom of the column to aspirate medium and 2 times 400 µl PBS washes. The dried beads were eluted with 20 µl of Citrate -NaOH pH 2.7 and the eluate was neutralized with 6 µl of 1M Tris-HCl pH 9.0. 10 µl 3x sample buffer plus DTT was added, the sample was boiled and 22 µl loaded for Western blot analysis. The membrane was sequentially stained with Ponceau-S, blocked in 4% milk, 0.5% Tween-20, then incubated with 1 µg/ml M2 in blocking buffer, washed, and revealed with goat anti-mouse-Ig-peroxidase (1/5000 in block buffer). ECL was used to illuminate the signal. Peroxidase activity was removed with azide, the membrane washed, and then probed again with donkey anti-human-peroxidase.

The western results of the co-immunoprecipitation show that only flag-muAPRIL is able to be co-immunoprecipitated by TACI(1-160)-Ig. The binding of TACI(1-160)-Ig to muAPRIL appears to be specific as none of the other 14 flag tagged soluble TNF family ligands were able to interact with TACI(1-160)-Ig.

### EXAMPLE 3

### Use of Derived TACI Sequence, and Antagonists and Agonists of TACI Binding and Activity, as Modifiers of Oncological and Neoplastic Disorders

Examples of the preparation and inoculation of transformed cells to assess tumor cell growth is described in Celis et al., CELL BIOLOGY, A LABORATORY HANDBOOK, Volume One, Academic Press, San Diego, CA. 1997. Tumor cells were derived from a variety of sources, including the extensive tumor cell banks maintained by ATCC (Bethesda, MD), immortalized cell lines, immortalized primary cell lines, stably transfected cell lines, tumor tissue derived from mammalian sources, including humans. Tissue source is a major determinant of choice of immunodeficient or normal animals (Celis *et al., ibid.*). In addition there are a wide variety of techniques for the induction of tumor growth in various animal models using carcinogenic or other insults.

Models which utilize tumor growth in immunodeficient mice as a means of readily determining the activity of novel ligands on tumor biology (Hahne et al. (1998) J. Exp. Med. 188:1185-1190) were developed. Both ligands and their antagonists were assayed in such systems, *e.g.,* Kashii et al. (1999) J. Immunol. 163:5358-5366; Zhai et al. (1999) FASEB J. 13:181-189). An agonist mAb to a TNF receptor family member (LTBR) profoundly affected tumor cell growth and survival was demonstrated (Browning et al. (1996) J. Exp. Med. 183:867-878).

Tumor cell lines were implanted in immunodeficient mice subcutaneously, and the growth rate of tumors in mice treated with TACI-Ig was similar to the growth rate of tumors in mice treated with approximately 5mg/kg/week CBE11, that is, much slower tumor growth as compared to the growth rate of mice given control treatments.

Another variation of these models was to use as a tumor source cells known to induce metastasis in immunodeficient or syngeneic animals. For example, the ATCC (Bethesda, MD) provided numerous human tumor lines with known metastatic potential to a variety of tissues, and these lines are utilized to examine the effect of TACI activity or antagonism on metastasis. These models have been and are now currently used (*e.g.,* by the NCI) to assess the potential for treatment of human patients.

### EXAMPLE 4

### Generation of Soluble TACI Receptors

To form a receptor inhibitor for use in man, the human receptor cDNA sequence of the extracellular domain of TACI was used (SEQ ID NO:2). With a human cDNA sequence, oligonucleotide primers were designed to PCR amplify the extracellular domain of the receptor in the absence of the transmembrane and intracellular domains. Typically, one of ordinary skill included most of the amino acids between the last disulfide linked "TNF domain" and the transmembrane domain. Alternatively, the amount of "stalk" region was varied to optimize the potency of the resultant soluble receptor. This amplified piece was engineered to include suitable restriction sites to allow cloning into various C-terminal Ig fusion chimera vectors. Alternatively, a stop signal at the 3' end was inserted to make a soluble form of the receptor without resorting to the use of an Ig fusion chimera approach. The resultant vectors were expressed in most systems used in biotechnology including yeast, insect cells, bacteria and mammalian cells and examples exist for all types of expression. Various human Fc domains were attached to optimize or eliminate FcR and complement interactions as desired. Alternatively, mutated forms of these Fc domains were used to selectively remove FcR or complement interactions or the attachment of N-linked sugars to the Fc domain which has certain advantages.

### EXAMPLE 5

### Screening for Inhibitors of the Receptor-Lisand Interaction

Using the receptor-Ig fusion protein, one screens either combinatorial libraries as screened for molecules that bind the receptor directly. These molecules are then tested in an ELISA formatted assay using the receptor-Ig fusion protein and a soluble form of the ligand for the ability to inhibit the receptor-ligand interaction. This ELISA is used directly to screen various natural product libraries, *etc.* for inhibitory compounds. The receptor is transfected into a cell line such as the HT29 line to form a biological assay (in this case cytotoxicity) that then form the screening assay to further demonstrate blocking.

### EXAMPLE 6

### In vivo Tumor Growth Inhibition

The number of tumor cells injected subcutaneously (s.c.) can be determined in titration studies prior to initiating work with antagonists. For example, the SW480 - colon adenocarcinoma solid tumor line and NIH 3T3 fibrosarcoma line, which grow aggressively, 8 x 10⁵ cells and 5 x 10⁶ cells, respectively, can be implanted in nude mice. Dosing with control or TACI-Ig proteins can begin just prior to implantation, with subsequent doses every 7 days thereafter. The dose can be for example 100 µg/mouse. Tumor diameter is then measured using a micrometer, and the volume is calculated using the formula vol = 4/3πr³.

### EXAMPLE 7

### In vivo Tumor Growth Inhibition

The number of tumor cells injected s.c. into immunodeficient (nu/nu) mice was determined in previous dose response studies. For the studies using HT29, 1 x 10⁶ cells were implanted per mouse. HT29 is derived from a human colon adenocarcinoma, and has similar growth characteristics to some other human colon adenocarcinoma lines (eg., SW480) such as rapid tumor formation, and rapid tumor growth. Mice implanted with HT29 cells were treated on the day of implantation, and every week thereafter, with 100ugs hTACI(1-114)-Ig given intraperitoneally (i.p.). Negative controls included the irrelevant proteins polyclonal bIgG and mAb MOPC21. Positive controls included BCMA-Ig, another inhibitor of APRIL binding, and CBE11, a mAb to the mLTb-R which is known to slow adenocarcinoma tumor growth (Browning et al. (1996) J. Exp. Med. 183:867-878). Tumor diameter was measured using a micrometer, and the volume was calculated using the formula vol = 4/3πR³.

For studies with the lung carcinoma A549 we implanted 1 x 10⁶ cells/mouse. Mice were treated with 100 µg TACI(1-114)-Ig, 100 µg BCMA-Ig, 100 µg hIgG, or 100 µl PBS starting on the day of implantation and treated weekly thereafter. Tumor diameter was measured using a micrometer, and the volume was calculated using the formula vol = 4/3πR³.

The results showing tumor growth inhibition of the HT29 colon adenocarcinoma are shown in Figure 11. The results showing tumor growth inhibition of the A549 lung carcinoma are shown in Figure 12. In both experiments, significant slowing of tumor growth was achieved. Since tumor size and survival are directly linked in these models, TACI-Ig treatment also impacted animal survival. For example, 95 days after tumor implantation, 50% of the hIgG treated mice were scored as terminal, compared to only 12.5% of the TACI-Ig treated mice. This represents a 4 fold increase in survival at this end stage time point.

### EXAMPLE 8

### hTACI(1-114)-Ig and hTACI(32-114)-Ig bind to surface localized APRIL

This example shows titratable binding of TACI-Ig fusion proteins a stable cell line expressing muAPRIL on their surface. Either 25 µl or 5 µl of conditioned media from 293EBNA cells transiently transfected with plasmids expressing hTACI(1-114)-Ig, hTACI(32-114) or hFN14-Fc were diluted in FACS buffer to a final volume of 50 µl and incubated for 1 hour on ice with 2.5 x 10⁵ 293 cells stably expressing the receptor binding domain of muAPRIL on their surface. After washing with FACS buffer, cells were then incubated with anti-human IgG-PE (1:100 dilution, Jackson ImmunoResearch) for 30 minutes on ice. Cells were again washed, fixed in 1% paraformaldehyde and analyzed by FACS. Figure 13 shows an FL2 shift indicating titratable staining of surface muAPRIL by hTACI(1-114)-Ig and hTACI(32-114)-Ig. No staining was observed with the second step only control. No staining was observed with either dilution of a control-Ig, FN14-Ig fusion protein, both of which co-migrate with the second step only histogram.

### EXAMPLE 9

### Binding of murine and human APRIL to hTACI(32-114)-Ig in ELISA format

ELISA plates (Coming) were coated with 5 ug/ml hTACI(32-114)-Ig or mLTbR-Ig control in bicarbonate buffer pH = 9.6 overnight, then washed in PBS/0.05% Tween-20 solution before blocking for 2 h at 37°C with PBS/2% bovine serum albumin (BSA). APRIL protein preparations were added in the range of 0.0048 to 3 µg/ml, diluted in PBS/2% BSA. Detection of specific APRIL binding was with rabbit anti-murine APRIL antisera (R1532) and donkey anti-rabbit HRP (Jackson Immunoresearch) or with HRP-coupled anti FLAG mAb M2 (Kodak). Enzymatic development was done with TMA and H₂O₂ and stopped with H₂SO₄, following standard protocols. The developed yellow stain was read at 450 nm on a plate reader. The results are shown in Figure 14.

### EXAMPLE 10

### Preferential Expression of APRIL in Tumor Samples

APRIL mRNA is preferentially expressed in tumor samples. Analysis of the cDNA libraries collected by Incyte Inc., and shown in Table I, demonstrates that APRIL is widely expressed in numerous solid tumor and neoplastic samples, but much less often expressed in normal tissue samples. Expression in diseased tissue is occasionally prominent.

**Table I**

| **EXPRESSION OF APRIL IN TISSUES (INCYTE)** | | |
|---|---|---|
| **PROSTATE/BLADDER** | No. + | **DISTRIBUTION** |
| Normal | 5 | |
| Tumor | 11 | adenocarcinoma |

| **COLON** | | |
|---|---|---|
| Normal | 0 | |
| Tumor | 6 | adenocarcinoma |
| Diseased | 4 | 2 Crohn's Disease |
| | | 1 ulcerative colitis |
| | | 1 polyp |

| **BREAST** | | |
|---|---|---|
| Normal | 1 | normal epithelium |
| Tumor | 11 | 5 adenocarcinoma |
| | | 3 ductal carcinoma |
| | | 1 lobule carcinoma |
| | | 2 unspecified |

| **PANCREAS** | | |
|---|---|---|
| Normal | 0 | |
| Tumor | 5 | 1 anaplastic carcinoma |
| | | 4 adenocarcinoma |

| **LUNG** | | |
|---|---|---|
| Normal | 2 | 4 adenocarcinoma |
| Tumor | 12 | 4 adenocarcinoma |
| | | 3 squamous cell carcinoma |
| | | 3 carcinomoid |
| | | spindle cell |
| | | endobonchial |
| | | neuroendocrine |
| | | 2 metastases |
| Diseased | 7 | |

| **UTERUS/OVARY** | | |
|---|---|---|
| Normal | 0 | |
| Tumor | 13 | 3 cystadenocarcinoma |
| | | 2 endometrial tumors |
| | | 4 leiomyomata |
| | | 2 adenocarcinoma |
| | | 1 papillary carcinoma |
| | | 1 metastases |
| Diseased | 1 | 1 ovarian cyst |

| **Other Normal Tissues** | | |
|---|---|---|
| MO/DC | 10 | |
| **Endothelial** | 5 | 2 arterial endothelium |
| | | 1 dermal endothelium |
| | | 1 arterial smooth muscle |
| | | 1 arterial plaque |
| **Other Hyperplastic Sites** | 6 | 2 lymphomas |
| | | 2 lymphoid hyperplasia |
| | | 2 rheumatoid arthritis synovium |
| | | 1 hyperthyroid |
| | | 1 nasal polyp |

It is apparent to those skilled in the art that various modifications and variations are made in the methods of the invention without departing from the spirit or scope of the invention. Thus, it is intended that the present invention cover the modifications and variations of this invention provided that they come within the scope of the appended claims and their equivalents.

## Claims

1. A method of treating a mammal for a condition associated with undesired cell proliferation comprising administering to said mammal an effective amount of a TACI reagent that extends mean survival time of said mammal as compared to the mean survival time of said mammal in the absence of administering said TACI reagent, wherein said undesired cell proliferation comprises at least one solid tumor.

2. The method of claim 1, wherein said TACI reagent extends mean survival time of said mammal by about 15%.

3. The method of claim 1, wherein said TACI reagent extends mean survival time of said mammal by about 20%.

4. The method of claim 1, wherein said TACI reagent extends mean survival time of said mammal by about 25%.

5. The method of claims 1, 2, 3 or 4, wherein said TACI reagent is a fusion protein comprising at least two segments, wherein a first segment comprises a substantially pure TACI protein or polypeptide fragment thereof, and a second segment comprises an immunoglobulin polypeptide.

6. The method of claim 5, wherein the immunoglobulin polypeptide is an Fc domain.

7. The method of claim 6, wherein said Fc domain is an IgG Fc domain.

8. The method of claim 5 wherein said first segment of said fusion protein is selected from the group consisting of:
(a) the extracellular domain of a TACI polypeptide;
(b) amino acid residues 1-166 of SEQ ID NO:1;
(c) amino acid residues 1-160 of SEQ ID NO:1;
(d) amino acid residues 1-114 of SEQ ID NO:1; and
(e) amino acid residues 32-114 of SEQ ID NO: 1.

9. The method of claim 5 wherein said first segment of said fusion protein is selected from the group consisting of:
(a) a polypeptide having at least 80% amino acid sequence identity with the extracellular domain of a TACI polypeptide;
(b) a polypeptide having at least 80% amino acid sequence identity with amino acid residues 1-166 of SEQ ID NO:1;
(c) a polypeptide having at least 80% amino acid sequence identity with amino acid residues 1-160 of SEQ ID NO: 1;
(d) a polypeptide having at least 80% amino acid sequence identity with amino acid residues 1-114 of SEQ ID NO: 1; and
(e) a polypeptide having at least 80% amino acid sequence identity with amino acid residues 32-114 of SEQ ID NO:1.

10. The method of claims 1, 2, 3 or 4 wherein the said condition associated with undesired cell proliferation is a cancer selected from the group consisting of renal cell cancer, Kaposi's sarcoma, breast cancer, sarcoma, ovarian carcinoma, rectal cancer, throat cancer, melanoma, colon cancer, bladder cancer, mastocytoma, lung cancer, mammary adenocarcinoma, pharyngeal squamous cell carcinoma, gastrointestinal cancer, and stomach cancer.

11. The method of claims 1, 2, 3 or 4, wherein the mammal is selected from the group consisting of a human, a cow, a horse, a dog, a mouse, a rat, and a cat.

12. A method of reducing the size of a solid tumor located on or in a mammal comprising administering to said mammal an effective amount of a TACI reagent, wherein said reagent reduces the size of said solid tumor by about 10% or more.

13. The method of claim 12, wherein said TACI reagent reduces the size of said tumor by about 15% or more.

14. The method of claim 12, wherein said TACI reagent reduces the size of said tumor by about 20% or more.

15. The method of claim 12, wherein said TACI reagent reduces the size of said tumor by about 25% or more.

16. The method of claims 12, 13, 14 or 15 wherein said TACI reagent is a fusion protein comprising at least two segments, wherein a first segment comprises a substantially pure TACI protein or polypeptide fragment thereof, and a second segment comprises an immunoglobulin polypeptide.

17. The method of claim 16, wherein the immunoglobulin polypeptide is an Fc domain.

18. The method of claim 17, wherein said Fc domain is an IgG Fc domain.

19. The method of claim 16 wherein said first segment of said fusion protein is selected from the group consisting of:
(a) the extracellular domain of a TACI polypeptide;
(b) amino acid residues 1-166 of SEQ ID NO:1;
(c) amino acid residues 1-160 of SEQ ID NO:1;
(d) amino acid residues 1-114 of SEQ ID NO:1; and
(e) amino acid residues 32-114 of SEQ ID NO:1.

20. The method of claim 16 wherein said first segment of said fusion protein is selected from the group consisting of:
(a) a polypeptide having at least 80% amino acid sequence identity with the extracellular domain of a TACI polypeptide;
(b) a polypeptide having at least 80% amino acid sequence identity with amino acid residues 1-166 of SEQ ID NO:1;
(c) a polypeptide having at least 80% amino acid sequence identity with amino acid residues 1-160 of SEQ ID NO:1;
(d) a polypeptide having at least 80% amino acid sequence identity with amino acid residues 1-114 of SEQ ID NO:1; and
(e) a polypeptide having at least 80% amino acid sequence identity with amino acid residues 32-114 of SEQ ID NO:1.


### Annex to the application documents - subsequently filed sequences listing

SEQUENCE LISTING

<110> Biogen Idec MA Inc.
      Apoxis SA

<120> USE OF TACI AS AN ANTI-TUMOR AGENT

<130> BIO11752PCTEPD1

<140> EP 06 118 875.1
<141> 2006-08-14

<150> EP 01 927 474.5
<151> 2001-04-27

<150> PCT/US01/40626
<151> 2001-04-27

<150> 60/199,946
<151> 2000-04-27

<160> 17

<170> PatentIn Ver. 3.3

<210> 1
<211> 293
<212> PRT
<213> Homo sapiens

<400> 1

<210> 2
<211> 882
<212> DNA
<213> Homo sapiens

<220>
<221> CDS
<222> (1)..(879)

<400> 2
atg agt ggc ctg ggc cgg agc agg cga ggt ggc cgg agc cgt gtg gac      48
cag gag gag cgc ttt cca cag ggc ctg tgg acg ggg gtg gct atg aga      96
tcc tgc ccc gaa gag cag tac tgg gat cct ctg ctg ggt acc tgc atg      144
tcc tgc aaa acc att tgc aac cat cag agc cag cgc acc tgt gca gcc      192
ttc tgc agg tca ctc agc tgc cgc aag gag caa ggc aag ttc tat gac      240
cat ctc ctg agg gac tgc atc agc tgt gcc tcc atc tgt gga cag cac      288
cct aag caa tgt gca tac ttc tgt gag aac aag ctc agg agc cca gtg      336
aac ctt cca cca gag ctc agg aga cag cgg agt gga gaa gtt gaa aac      384
aat tca gac aac tcg gga agg tac caa gga ttg gag cac aga ggc tca      432
gaa gca agt cca gct ctc ccg ggg ctg aag ctg agt gca gat cag gtg      480
gcc ctg gtc tac agc acg ctg ggg ctc tgc ctg tgt gcc gtc ctc tgc     528
tgc ttc ctg gtg gcg gtg gcc tgc ttc ctc aag aag agg ggg gat ccc     576
tgc tcc tgc cag ccc cgc tca agg ccc cgt caa agt ccg gcc aag tct     624
tcc cag gat cac gcg atg gaa gcc ggc agc cct gtg agc aca tcc ccc     672
gag cca gtg gag acc tgc agc ttc tgc ttc cct gag tgc agg gcg ccc     720
acg cag gag agc gca gtc acg cct ggg acc ccc gac ccc act tgt gct     768
gga agg tgg ggg tgc cac acc agg acc aca gtc ctg cag cct tgc cca     816
cac atc cca gac agt ggc ctt ggc att gtg tgt gtg cct gcc cag gag     864
ggg ggc cca ggt gca taa                                             882

<210> 3
<211> 249
<212> PRT
<213> Mus musculus

<400> 3
Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Glu Gln Lys Leu Ile Ser Glu Glu Asp Leu Asn
                85                  90                  95

Gly Gly Gly Gly Ser Lys Glu Leu Ala Val Leu Thr Gln Lys His Lys
            100                 105                 110

Lys Lys His Ser Val Leu His Leu Val Pro Val Asn Ile Thr Ser Lys
        115                 120                 125

Asp Ser Asp Val Thr Glu Val Met Trp Gln Pro Val Leu Arg Arg Gly
    130                 135                 140

Arg Gly Leu Glu Ala Gln Gly Asp Ile Val Arg Val Trp Asp Thr Gly
145                 150                 155                 160

Ile Tyr Leu Leu Tyr Ser Gln Val Leu Phe His Asp Val Thr Phe Thr
                165                 170                 175

Met Gly Gln Val Val Ser Arg Glu Gly Gln Gly Arg Arg Glu Thr Leu
            180                 185                 190

Phe Arg Cys Ile Arg Ser Met Pro Ser Asp Pro Asp Arg Ala Tyr Asn
        195                 200                 205

Ser Cys Tyr Ser Ala Gly Val Phe His Leu His Gln Gly Asp Ile Ile
    210                 215                 220

Thr Val Lys Ile Pro Arg Ala Asn Ala Lys Leu Ser Leu Ser Pro His
225                 230                 235                 240

Gly Thr Phe Leu Gly Phe Val Lys Leu
                245


<210> 4
<211> 788
<212> DNA
<213> Mus musculus

<220>
<221> CDS
<222> (11)..(757)

<400> 4
gatccaaacg atg aga ttt cct tca att ttt act gca gtt tta ttc gca      49
           Met Arg Phe Pro Ser Ile Phe Thr Ala Val Leu Phe Ala
           1               5                   10

gca tcc tcc gca tta gct gct cca gtc aac act aca aca gaa gat gaa      97
Ala Ser Ser Ala Leu Ala Ala Pro Val Asn Thr Thr Thr Glu Asp Glu
    15                  20                  25

acg gca caa att ccg gct gaa gct gtc atc ggt tac tca gat tta gaa      145
Thr Ala Gln Ile Pro Ala Glu Ala Val Ile Gly Tyr Ser Asp Leu Glu
30                  35                  40                  45

ggg gat ttc gat gtt gct gtt ttg cca ttt tcc aac agc aca aat aac      193
Gly Asp Phe Asp Val Ala Val Leu Pro Phe Ser Asn Ser Thr Asn Asn
                50                  55                  60

ggg tta ttg ttt ata aat act act att gcc agc att gct gct aaa gaa      241
Gly Leu Leu Phe Ile Asn Thr Thr Ile Ala Ser Ile Ala Ala Lys Glu
            65                  70                  75

gaa ggg gta tct ctc gag aaa aga gaa caa aaa ctc att tct gag gaa      289
Glu Gly Val Ser Leu Glu Lys Arg Glu Gln Lys Leu Ile Ser Glu Glu
        80                  85                  90

gat ctg aat ggt ggc ggt ggg tcc aaa gag ctc gca gta ctc acc cag      337
Asp Leu Asn Gly Gly Gly Gly Ser Lys Glu Leu Ala Val Leu Thr Gln
    95                  100                 105

aag cac aag aag aag cac tca gtc ctg cat ctt gtt cca gtt aac att      385
Lys His Lys Lys Lys His Ser Val Leu His Leu Val Pro Val Asn Ile
110                 115                 120                 125

acc tcc aag gac tct gac gtg aca gag gtg atg tgg caa cca gta ctt      433

Thr Ser Lys Asp Ser Asp Val Thr Glu Val Met Trp Gln Pro Val Leu
                130                 135                 140

agg cgt ggg aga ggc ctg gag gcc cag gga gac att gta cga gtc tgg     481
Arg Arg Gly Arg Gly Leu Glu Ala Gln Gly Asp Ile Val Arg Val Trp
            145                 150                 155

gac act gga att tat ctg ctc tat agt cag gtc ctg ttt cat gat gtg     529
Asp Thr Gly Ile Tyr Leu Leu Tyr Ser Gln Val Leu Phe His Asp Val
        160                 165                 170

act ttc aca atg ggt cag gtg gta tct cgg gaa gga caa ggg aga aga     577
Thr Phe Thr Met Gly Gln Val Val Ser Arg Glu Gly Gln Gly Arg Arg
    175                 180                 185

gaa act cta ttc cga tgt atc aga agt atg cct tct gat cct gac cgt     625
Glu Thr Leu Phe Arg Cys Ile Arg Ser Met Pro Ser Asp Pro Asp Arg
190                 195                 200                 205

gcc tac aat agc tgc tac agt gca ggt gtc ttt cat tta cat caa ggg     673
Ala Tyr Asn Ser Cys Tyr Ser Ala Gly Val Phe His Leu His Gln Gly
                210                 215                 220

gat att atc act gtc aaa att cca cgg gca aac gca aaa ctt agc ctt     721
Asp Ile Ile Thr Val Lys Ile Pro Arg Ala Asn Ala Lys Leu Ser Leu
            225                 230                 235

tct ccg cat gga aca ttc ctg ggg ttt gtg aaa cta tgagcggccg          767
Ser Pro His Gly Thr Phe Leu Gly Phe Val Lys Leu
        240                 245

cgaattaatt cgccttagac a                                             788

<210> 5
<211> 1101
<212> DNA
<213> Homo sapiens

<220>
<221> CDS
<222> (1)..(1101)

<400> 5
atg gag aca gac aca ctc ctg tta tgg gtg ctg ctg ctc tgg gtt cca     48
Met Glu Thr Asp Thr Leu Leu Leu Trp Val Leu Leu Leu Trp Val Pro
1               5                   10                  15

ggt tcc act ggt gac gtc acg atg agt ggc ctg ggc cgg agc agg cga     96
Gly Ser Thr Gly Asp Val Thr Met Ser Gly Leu Gly Arg Ser Arg Arg
            20                  25                  30

ggt ggc cgg agc cgt gtg gac cag gag gag cgc ttt cca cag ggc ctg     144
Gly Gly Arg Ser Arg Val Asp Gln Glu Glu Arg Phe Pro Gln Gly Leu
        35                  40                  45

tgg aca ggg gtg gct atg aga tcc tgc ccc gaa gag cag tac tgg gat     192
Trp Thr Gly Val Ala Met Arg Ser Cys Pro Glu Glu Gln Tyr Trp Asp
    50                  55                  60

cct ctg ctg ggt acc tgc atg tcc tgc aaa acc att tgc aac cat cag     240
Pro Leu Leu Gly Thr Cys Met Ser Cys Lys Thr Ile Cys Asn His Gln
65                  70                  75                  80

agc cag cgc acc tgt gca gcc ttc tgc agg tca ctc agc tgc cgc aag     288
Ser Gln Arg Thr Cys Ala Ala Phe Cys Arg Ser Leu Ser Cys Arg Lys
                85                  90                  95

gag caa ggc aag ttc tat gac cat ctc ctg agg gac tgc atc agc tgt     336
Glu Gln Gly Lys Phe Tyr Asp His Leu Leu Arg Asp Cys Ile Ser Cys
            100                 105                 110

gcc tcc atc tgt gga cag cac cct aag caa tgt gca tac ttc tgt gag     384
Ala Ser Ile Cys Gly Gln His Pro Lys Gln Cys Ala Tyr Phe Cys Glu
        115                 120                 125

aac aag ctc agg agc cca gtg aac ctt cca gtc gac aaa act cac aca     432
Asn Lys Leu Arg Ser Pro Val Asn Leu Pro Val Asp Lys Thr His Thr
    130                 135                 140

tgc cca ccg tgc cca gca cct gaa ctc ctg ggg gga ccg tca gtc ttc     480
Cys Pro Pro Cys Pro Ala Pro Glu Leu Leu Gly Gly Pro Ser Val Phe
145                 150                 155                 160

ctc ttc ccc cca aaa ccc aag gac acc ctc atg atc tcc cgg acc cct     528
Leu Phe Pro Pro Lys Pro Lys Asp Thr Leu Met Ile Ser Arg Thr Pro
                165                 170                 175

gag gtc aca tgc gtg gtg gtg gac gtg agc cac gaa gac cct gag gtc     576
Glu Val Thr Cys Val Val Val Asp Val Ser His Glu Asp Pro Glu Val
            180                 185                 190

aag ttc aac tgg tac gtg gac ggc gtg gag gtg cat aat gcc aag aca     624
Lys Phe Asn Trp Tyr Val Asp Gly Val Glu Val His Asn Ala Lys Thr
        195                 200                 205

aag ccg cgg gag gag cag tac aac agc acg tac cgt gtg gtc agc gtc     672
Lys Pro Arg Glu Glu Gln Tyr Asn Ser Thr Tyr Arg Val Val Ser Val
    210                 215                 220

ctc acc gtc ctg cac cag gac tgg ctg aat ggc aag gag tac aag tgc     720
Leu Thr Val Leu His Gln Asp Trp Leu Asn Gly Lys Glu Tyr Lys Cys
225                 230                 235                 240

aag gtc tcc aac aaa gcc ctc cca gcc ccc atc gag aaa acc atc tcc     768
Lys Val Ser Asn Lys Ala Leu Pro Ala Pro Ile Glu Lys Thr Ile Ser
                245                 250                 255

aaa gcc aaa ggg cag ccc cga gaa cca cag gtg tac acc ctg ccc cca     816
Lys Ala Lys Gly Gln Pro Arg Glu Pro Gln Val Tyr Thr Leu Pro Pro
            260                 265                 270

tcc cgg gat gag ctg acc aag aac cag gtc agc ctg acc tgc ctg gtc     864
Ser Arg Asp Glu Leu Thr Lys Asn Gln Val Ser Leu Thr Cys Leu Val
        275                 280                 285

aaa ggc ttc tat ccc agc gac atc gcc gtg gag tgg gag agc aat ggg     912
Lys Gly Phe Tyr Pro Ser Asp Ile Ala Val Glu Trp Glu Ser Asn Gly
    290                 295                 300

cag ccg gag aac aac tac aag acc acg cct ccc gtg ttg gac tcc gac     960
Gln Pro Glu Asn Asn Tyr Lys Thr Thr Pro Pro Val Leu Asp Ser Asp
305                 310                 315                 320

ggc tcc ttc ttc ctc tac agc aag ctc acc gtg gac aag agc agg tgg     1008
Gly Ser Phe Phe Leu Tyr Ser Lys Leu Thr Val Asp Lys Ser Arg Trp
                325                 330                 335

cag cag ggg aac gtc ttc tca tgc tcc gtg atg cat gag gct ctg cac     1056
Gln Gln Gly Asn Val Phe Ser Cys Ser Val Met His Glu Ala Leu His
            340                 345                 350

aac cac tac acg cag aag agc ctc tcc ctg tct ccc ggg aaa tga         1101
Asn His Tyr Thr Gln Lys Ser Leu Ser Leu Ser Pro Gly Lys
        355                 360                 365

<210> 6
<211> 366
<212> PRT
<213> Homo sapiens

<400> 6
Met Glu Thr Asp Thr Leu Leu Leu Trp Val Leu Leu Leu Trp Val Pro
1               5                   10                  15

Gly Ser Thr Gly Asp Val Thr Met Ser Gly Leu Gly Arg Ser Arg Arg
            20                  25                  30

Gly Gly Arg Ser Arg Val Asp Gln Glu Glu Arg Phe Pro Gln Gly Leu
        35                  40                  45

Trp Thr Gly Val Ala Met Arg Ser Cys Pro Glu Glu Gln Tyr Trp Asp
    50                  55                  60

Pro Leu Leu Gly Thr Cys Met Ser Cys Lys Thr Ile Cys Asn His Gln
65                  70                  75                  80

Ser Gln Arg Thr Cys Ala Ala Phe Cys Arg Ser Leu Ser Cys Arg Lys
                85                  90                  95

Glu Gln Gly Lys Phe Tyr Asp His Leu Leu Arg Asp Cys Ile Ser Cys
            100                 105                 110

Ala Ser Ile Cys Gly Gln His Pro Lys Gln Cys Ala Tyr Phe Cys Glu
        115                 120                 125

Asn Lys Leu Arg Ser Pro Val Asn Leu Pro Val Asp Lys Thr His Thr
    130                 135                 140

Cys Pro Pro Cys Pro Ala Pro Glu Leu Leu Gly Gly Pro Ser Val Phe
145                 150                 155                 160

Leu Phe Pro Pro Lys Pro Lys Asp Thr Leu Met Ile Ser Arg Thr Pro
                165                 170                 175

Glu Val Thr Cys Val Val Val Asp Val Ser His Glu Asp Pro Glu Val
            180                 185                 190

Lys Phe Asn Trp Tyr Val Asp Gly Val Glu Val His Asn Ala Lys Thr
        195                 200                 205

Lys Pro Arg Glu Glu Gln Tyr Asn Ser Thr Tyr Arg Val Val Ser Val
    210                 215                 220

Leu Thr Val Leu His Gln Asp Trp Leu Asn Gly Lys Glu Tyr Lys Cys
225                 230                 235                 240

Lys Val Ser Asn Lys Ala Leu Pro Ala Pro Ile Glu Lys Thr Ile Ser
                245                 250                 255

Lys Ala Lys Gly Gln Pro Arg Glu Pro Gln Val Tyr Thr Leu Pro Pro
            260                 265                 270

Ser Arg Asp Glu Leu Thr Lys Asn Gln Val Ser Leu Thr Cys Leu Val
        275                 280                 285

Lys Gly Phe Tyr Pro Ser Asp Ile Ala Val Glu Trp Glu Ser Asn Gly
    290                 295                 300

Gln Pro Glu Asn Asn Tyr Lys Thr Thr Pro Pro Val Leu Asp Ser Asp
305                 310                 315                 320

Gly Ser Phe Phe Leu Tyr Ser Lys Leu Thr Val Asp Lys Ser Arg Trp
                325                 330                 335

Gln Gln Gly Asn Val Phe Ser Cys Ser Val Met His Glu Ala Leu His
            340                 345                 350

Asn His Tyr Thr Gln Lys Ser Leu Ser Leu Ser Pro Gly Lys
        355                 360                 365

<210> 7
<211> 1005
<212> DNA
<213> Homo sapiens

<220>
<221> CDS
<222> (1)..(1002)

<400> 7
atg gag aca gac aca ctc ctg tta tgg gtg ctg ctg ctc tgg gtt cca      48
Met Glu Thr Asp Thr Leu Leu Leu Trp Val Leu Leu Leu Trp Val Pro
  1               5                  10                  15

ggt tcc act ggt gac gtc aga tcc tgc ccc gaa gag cag tac tgg gat      96
Gly Ser Thr Gly Asp Val Arg Ser Cys Pro Glu Glu Gln Tyr Trp Asp
             20                  25                  30

cct ctg ctg ggt acc tgc atg tcc tgc aaa acc att tgc aac cat cag     144
Pro Leu Leu Gly Thr Cys Met Ser Cys Lys Thr Ile Cys Asn His Gln
         35                  40                  45

agc cag cgc acc tgt gca gcc ttc tgc agg tca ctc agc tgc cgc aag     192
Ser Gln Arg Thr Cys Ala Ala Phe Cys Arg Ser Leu Ser Cys Arg Lys
     50                  55                  60

gag caa ggc aag ttc tat gac cat ctc ctg agg gac tgc atc agc tgt     240
Glu Gln Gly Lys Phe Tyr Asp His Leu Leu Arg Asp Cys Ile Ser Cys
 65                  70                  75                  80

gcc tcc atc tgt gga cag cac cct aag caa tgt gca tac ttc tgt gag     288
Ala Ser Ile Cys Gly Gln His Pro Lys Gln Cys Ala Tyr Phe Cys Glu
                 85                  90                  95

aac aag ctc agg agc cca gtg aac ctt cca gtc gac aaa act cac aca     336
Asn Lys Leu Arg Ser Pro Val Asn Leu Pro Val Asp Lys Thr His Thr
            100                 105                 110

tgc cca ccg tgc cca gca cct gaa ctc ctg ggg gga ccg tca gtc ttc     384
Cys Pro Pro Cys Pro Ala Pro Glu Leu Leu Gly Gly Pro Ser Val Phe
        115                 120                 125

ctc ttc ccc cca aaa ccc aag gac acc ctc atg atc tcc cgg acc cct     432
Leu Phe Pro Pro Lys Pro Lys Asp Thr Leu Met Ile Ser Arg Thr Pro
    130                 135                 140

gag gtc aca tgc gtg gtg gtg gac gtg agc cac gaa gac cct gag gtc     480
Glu Val Thr Cys Val Val Val Asp Val Ser His Glu Asp Pro Glu Val
145                 150                 155                 160

aag ttc aac tgg tac gtg gac ggc gtg gag gtg cat aat gcc aag aca     528
Lys Phe Asn Trp Tyr Val Asp Gly Val Glu Val His Asn Ala Lys Thr
                165                 170                 175

aag ccg cgg gag gag cag tac aac agc acg tac cgt gtg gtc agc gtc     576
Lys Pro Arg Glu Glu Gln Tyr Asn Ser Thr Tyr Arg Val Val Ser Val
            180                 185                 190

ctc acc gtc ctg cac cag gac tgg ctg aat ggc aag gag tac aag tgc     624
Leu Thr Val Leu His Gln Asp Trp Leu Asn Gly Lys Glu Tyr Lys Cys

195                 200                 205

aag gtc tcc aac aaa gcc ctc cca gcc ccc atc gag aaa acc atc tcc     672
Lys Val Ser Asn Lys Ala Leu Pro Ala Pro Ile Glu Lys Thr Ile Ser
    210                 215                 220

aaa gcc aaa ggg cag ccc cga gaa cca cag gtg tac acc ctg ccc cca     720
Lys Ala Lys Gly Gln Pro Arg Glu Pro Gln Val Tyr Thr Leu Pro Pro
225                 230                 235                 240

tcc cgg gat gag ctg acc aag aac cag gtc agc ctg acc tgc ctg gtc     768
Ser Arg Asp Glu Leu Thr Lys Asn Gln Val Ser Leu Thr Cys Leu Val
                245                 250                 255

aaa ggc ttc tat ccc agc gac atc gcc gtg gag tgg gag agc aat ggg     816
Lys Gly Phe Tyr Pro Ser Asp Ile Ala Val Glu Trp Glu Ser Asn Gly
            260                 265                 270

cag ccg gag aac aac tac aag acc acg cct ccc gtg ttg gac tcc gac     864
Gln Pro Glu Asn Asn Tyr Lys Thr Thr Pro Pro Val Leu Asp Ser Asp
        275                 280                 285

ggc tcc ttc ttc ctc tac agc aag ctc acc gtg gac aag agc agg tgg     912
Gly Ser Phe Phe Leu Tyr Ser Lys Leu Thr Val Asp Lys Ser Arg Trp
    290                 295                 300

cag cag ggg aac gtc ttc tca tgc tcc gtg atg cat gag gct ctg cac     960
Gln Gln Gly Asn Val Phe Ser Cys Ser Val Met His Glu Ala Leu His
305                 310                 315                 320

aac cac tac acg cag aag agc ctc tcc ctg tct ccc ggg aaa tga         1005
Asn His Tyr Thr Gln Lys Ser Leu Ser Leu Ser Pro Gly Lys
                325                 330


<210> 8
<211> 334
<212> PRT
<213> Homo sapiens

<400> 8
Met Glu Thr Asp Thr Leu Leu Leu Trp Val Leu Leu Leu Trp Val Pro
1               5                   10                  15

Gly Ser Thr Gly Asp Val Arg Ser Cys Pro Glu Glu Gln Tyr Trp Asp
            20                  25                  30

Pro Leu Leu Gly Thr Cys Met Ser Cys Lys Thr Ile Cys Asn His Gln
        35                  40                  45

Ser Gln Arg Thr Cys Ala Ala Phe Cys Arg Ser Leu Ser Cys Arg Lys
    50                  55                  60

Glu Gln Gly Lys Phe Tyr Asp His Leu Leu Arg Asp Cys Ile Ser Cys
65                  70                  75                  80

Ala Ser Ile Cys Gly Gln His Pro Lys Gln Cys Ala Tyr Phe Cys Glu
                85                  90                  95

Asn Lys Leu Arg Ser Pro Val Asn Leu Pro Val Asp Lys Thr His Thr
            100                 105                 110

Cys Pro Pro Cys Pro Ala Pro Glu Leu Leu Gly Gly Pro Ser Val Phe
        115                 120                 125

Leu Phe Pro Pro Lys Pro Lys Asp Thr Leu Met Ile Ser Arg Thr Pro
    130                 135                 140

Glu Val Thr Cys Val Val Val Asp Val Ser His Glu Asp Pro Glu Val

145                 150                 155                 160

Lys Phe Asn Trp Tyr Val Asp Gly Val Glu Val His Asn Ala Lys Thr
                165                 170                 175

Lys Pro Arg Glu Glu Gln Tyr Asn Ser Thr Tyr Arg Val Val Ser Val
            180                 185                 190

Leu Thr Val Leu His Gln Asp Trp Leu Asn Gly Lys Glu Tyr Lys Cys
        195                 200                 205

Lys Val Ser Asn Lys Ala Leu Pro Ala Pro Ile Glu Lys Thr Ile Ser
    210                 215                 220

Lys Ala Lys Gly Gln Pro Arg Glu Pro Gln Val Tyr Thr Leu Pro Pro
225                 230                 235                 240

Ser Arg Asp Glu Leu Thr Lys Asn Gln Val Ser Leu Thr Cys Leu Val
                245                 250                 255

Lys Gly Phe Tyr Pro Ser Asp Ile Ala Val Glu Trp Glu Ser Asn Gly
            260                 265                 270

Gln Pro Glu Asn Asn Tyr Lys Thr Thr Pro Pro Val Leu Asp Ser Asp
        275                 280                 285

Gly Ser Phe Phe Leu Tyr Ser Lys Leu Thr Val Asp Lys Ser Arg Trp
    290                 295                 300

Gln Gln Gly Asn Val Phe Ser Cys Ser Val Met His Glu Ala Leu His
305                 310                 315                 320

Asn His Tyr Thr Gln Lys Ser Leu Ser Leu Ser Pro Gly Lys
                325                 330

<210> 9
<211> 531
<212> DNA
<213> Homo sapiens

<220>
<221> CDS
<222> (1)..(531)

<400> 9
atg gct atc atc tac ctc atc ctc ctg ttc acc gct gtg cgg ggc gat      48
Met Ala Ile Ile Tyr Leu Ile Leu Leu Phe Thr Ala Val Arg Gly Asp
1               5                   10                  15

tac aaa gac gat gac gat aaa gga ccc gga cag gtg cag ctg cag gca      96
Tyr Lys Asp Asp Asp Asp Lys Gly Pro Gly Gln Val Gln Leu Gln Ala
            20                  25                  30

gtg ctc acc caa aaa cag aag aag cag cac tct gtc ctg cac ctg gtt      144
Val Leu Thr Gln Lys Gln Lys Lys Gln His Ser Val Leu His Leu Val
        35                  40                  45

ccc att aac gcc acc tcc aag gat gac tcc gat gtg aca gag gtg atg      192
Pro Ile Asn Ala Thr Ser Lys Asp Asp Ser Asp Val Thr Glu Val Met
    50                  55                  60

tgg caa cca gct ctt agg cgt ggg aga ggc cta cag gcc caa gga tat      240
Trp Gln Pro Ala Leu Arg Arg Gly Arg Gly Leu Gln Ala Gln Gly Tyr
65                  70                  75                  80

ggt gtc cga atc cag gat gct gga gtt tat ctg ctg tat agc cag gtc      288
Gly Val Arg Ile Gln Asp Ala Gly Val Tyr Leu Leu Tyr Ser Gln Val

                        85                  90                  95

ctg ttt caa gac gtg act ttc acc atg ggt cag gtg gtg tct cga gaa     336
Leu Phe Gln Asp Val Thr Phe Thr Met Gly Gln Val Val Ser Arg Glu
            100                 105                 110

ggc caa gga agg cag gag act cta ttc cga tgt ata aga agt atg ccc     384
Gly Gln Gly Arg Gln Glu Thr Leu Phe Arg Cys Ile Arg Ser Met Pro
        115                 120                 125

tcc cac ccg gac cgg gcc tac aac agc tgc tat agc gca ggt gtc ttc     432
Ser His Pro Asp Arg Ala Tyr Asn Ser Cys Tyr Ser Ala Gly Val Phe
    130                 135                 140

cat tta cac caa ggg gat att ctg agt gtc ata att ccc cgg gca agg     480
His Leu His Gln Gly Asp Ile Leu Ser Val Ile Ile Pro Arg Ala Arg
145                 150                 155                 160

gcg aaa ctt aac ctc tct cca cat gga acc ttc ctg ggg ttt gtg aaa     528
Ala Lys Leu Asn Leu Ser Pro His Gly Thr Phe Leu Gly Phe Val Lys
                165                 170                 175

ctg                                                                 531
Leu


<210> 10
<211> 177
<212> PRT
<213> Homo sapiens

<400> 10
Met Ala Ile Ile Tyr Leu Ile Leu Leu Phe Thr Ala Val Arg Gly Asp
1               5                   10                  15

Tyr Lys Asp Asp Asp Asp Lys Gly Pro Gly Gln Val Gln Leu Gln Ala
            20                  25                  30

Val Leu Thr Gln Lys Gln Lys Lys Gln His Ser Val Leu His Leu Val
        35                  40                  45

Pro Ile Asn Ala Thr Ser Lys Asp Asp Ser Asp Val Thr Glu Val Met
    50                  55                  60

Trp Gln Pro Ala Leu Arg Arg Gly Arg Gly Leu Gln Ala Gln Gly Tyr
65                  70                  75                  80

Gly Val Arg Ile Gln Asp Ala Gly Val Tyr Leu Leu Tyr Ser Gln Val
                85                  90                  95

Leu Phe Gln Asp Val Thr Phe Thr Met Gly Gln Val Val Ser Arg Glu
            100                 105                 110

Gly Gln Gly Arg Gln Glu Thr Leu Phe Arg Cys Ile Arg Ser Met Pro
        115                 120                 125

Ser His Pro Asp Arg Ala Tyr Asn Ser Cys Tyr Ser Ala Gly Val Phe
    130                 135                 140

His Leu His Gln Gly Asp Ile Leu Ser Val Ile Ile Pro Arg Ala Arg
145                 150                 155                 160

Ala Lys Leu Asn Leu Ser Pro His Gly Thr Phe Leu Gly Phe Val Lys
                165                 170                 175

Leu

<210> 11
<211> 1470
<212> DNA
<213> Homo sapiens

<220>
<221> CDS
<222> (119)..(1021)

<220>
<221> CDS
<222> (1025)..(1054)

<400> 11
acgcaaatgg gcggtaggcg tgtacggtgg gaggtctata taagcagagc tcgtttagtg 60

aaccgtcaga tctctagaag ctgggtacca gctgctagca agcttgctag cggccgcc 118

atg gac tac aaa gac gat gac gac aag agt ggc ctg ggc cgg agc agg 166
Met Asp Tyr Lys Asp Asp Asp Asp Lys Ser Gly Leu Gly Arg Ser Arg
1               5                   10                  15

cga ggt ggc cgg agc cgt gtg gac cag gag gag cgc ttt cca cag ggc 214
Arg Gly Gly Arg Ser Arg Val Asp Gln Glu Glu Arg Phe Pro Gln Gly
            20                  25                  30

ctg tgg acg ggg gtg gct atg aga tcc tgc ccc gaa gag cag tac tgg 262
Leu Trp Thr Gly Val Ala Met Arg Ser Cys Pro Glu Glu Gln Tyr Trp
        35                  40                  45

gat cct ctg ctg ggt acc tgc atg tcc tgc aaa acc att tgc aac cat 310
Asp Pro Leu Leu Gly Thr Cys Met Ser Cys Lys Thr Ile Cys Asn His
    50                  55                  60

cag agc cag cgc acc tgt gca gcc ttc tgc agg tca ctc agc tgc cgc 358
Gln Ser Gln Arg Thr Cys Ala Ala Phe Cys Arg Ser Leu Ser Cys Arg
65                  70                  75                  80

aag gag caa ggc aag ttc tat gac cat ctc ctg agg gac tgc atc agc 406
Lys Glu Gln Gly Lys Phe Tyr Asp His Leu Leu Arg Asp Cys Ile Ser
                85                  90                  95

tgt gcc tcc atc tgt gga cag cac cct aag caa tgt gca tac ttc tgt 454
Cys Ala Ser Ile Cys Gly Gln His Pro Lys Gln Cys Ala Tyr Phe Cys
            100                 105                 110

gag aac aag ctc agg agc cca gtg aac ctt cca cca gag ctc agg aga 502
Glu Asn Lys Leu Arg Ser Pro Val Asn Leu Pro Pro Glu Leu Arg Arg
        115                 120                 125

cag cgg agt gga gaa gtt gaa aac aat tca gac aac tcg gga agg tac 550
Gln Arg Ser Gly Glu Val Glu Asn Asn Ser Asp Asn Ser Gly Arg Tyr
    130                 135                 140

caa gga ttg gag cac aga ggc tca gaa gca agt cca gct ctc ccg ggg 598
Gln Gly Leu Glu His Arg Gly Ser Glu Ala Ser Pro Ala Leu Pro Gly
145                 150                 155                 160

ctg aag ctg agt gca gat cag gtg gcc ctg gtc tac agc acg ctg ggg 646
Leu Lys Leu Ser Ala Asp Gln Val Ala Leu Val Tyr Ser Thr Leu Gly
                165                 170                 175

ctc tgc ctg tgt gcc gtc ctc tgc tgc ttc ctg gtg gcg gtg gcc tgc 694
Leu Cys Leu Cys Ala Val Leu Cys Cys Phe Leu Val Ala Val Ala Cys
            180                 185                 190

ttc ctc aag aag agg ggg gat ccc tgc tcc tgc cag ccc cgc tca agg 742
Phe Leu Lys Lys Arg Gly Asp Pro Cys Ser Cys Gln Pro Arg Ser Arg

        195                 200                 205

ccc cgt caa agt ccg gcc aag tct tcc cag gat cac gcg atg gaa gcc 790
Pro Arg Gln Ser Pro Ala Lys Ser Ser Gln Asp His Ala Met Glu Ala
    210                 215                 220

ggc agc cct gtg agc aca tcc ccc gag cca gtg gag acc tgc agc ttc 838
Gly Ser Pro Val Ser Thr Ser Pro Glu Pro Val Glu Thr Cys Ser Phe
225                 230                 235                 240

tgc ttc cct gag tgc agg gcg ccc acg cag gag agc gca gtc acg cct 886
Cys Phe Pro Glu Cys Arg Ala Pro Thr Gln Glu Ser Ala Val Thr Pro
                245                 250                 255

ggg acc ccc gac ccc act tgt gct gga agg tgg ggg tgc cac acc agg 934
Gly Thr Pro Asp Pro Thr Cys Ala Gly Arg Trp Gly Cys His Thr Arg
            260                 265                 270

acc aca gtc ctg cag cct tgc cca cac atc cca gac agt ggc ctt ggc 982
Thr Thr Val Leu Gln Pro Cys Pro His Ile Pro Asp Ser Gly Leu Gly
        275                 280                 285

att gtg tgt gtg cct gcc cag gag ggg ggc cca ggt gca taa atg ggg 1030
Ile Val Cys Val Pro Ala Gln Glu Gly Gly Pro Gly Ala Met Gly
    290                 295                 300

gtc agc ggc cgc tcg agg ccg gca aggccggatc cagacatgat aagatacatt 1084
Val Ser Gly Arg Ser Arg Pro Ala
    305                 310

gatgagtttg gacaaaccac aactagaatg cagtgaaaaa aatgctttat ttgtgaaatt 1144

tgtgatgcta ttgctttatt tgtaaccatt ataagctgca ataaacaagt taacaacaac 1204

aattgcattc attttatgtt tcaggttcag ggggaggtgg ggaggttttt taaagcaagt 1264

aaaacctcta caaatgtggt atggctgatt atgatccggc tgcctcgcgc gtttcggtga 1324

tgacggtgaa aacctctgac acatgcagct cccggagacg gtcacagctt gtctgtaagc 1384

ggatgccggg agcagacaag cccgtcaggg cgcgtcagcg ggtgttggcg ggtgtcgggg 1444

cgcagccatg accggtcgac tctaga 1470

<210> 12
<211> 301
<212> PRT
<213> Homo sapiens

<400> 12
Met Asp Tyr Lys Asp Asp Asp Asp Lys Ser Gly Leu Gly Arg Ser Arg
1               5                   10                  15

Arg Gly Gly Arg Ser Arg Val Asp Gln Glu Glu Arg Phe Pro Gln Gly
            20                  25                  30

Leu Trp Thr Gly Val Ala Met Arg Ser Cys Pro Glu Glu Gln Tyr Trp
        35                  40                  45

Asp Pro Leu Leu Gly Thr Cys Met Ser Cys Lys Thr Ile Cys Asn His
    50                  55                  60

Gln Ser Gln Arg Thr Cys Ala Ala Phe Cys Arg Ser Leu Ser Cys Arg
65                  70                  75                  80

Lys Glu Gln Gly Lys Phe Tyr Asp His Leu Leu Arg Asp Cys Ile Ser
                85                  90                  95

Cys Ala Ser Ile Cys Gly Gln His Pro Lys Gln Cys Ala Tyr Phe Cys
            100                 105                 110

Glu Asn Lys Leu Arg Ser Pro Val Asn Leu Pro Pro Glu Leu Arg Arg
        115                 120                 125

Gln Arg Ser Gly Glu Val Glu Asn Asn Ser Asp Asn Ser Gly Arg Tyr
    130                 135                 140

Gln Gly Leu Glu His Arg Gly Ser Glu Ala Ser Pro Ala Leu Pro Gly
145                 150                 155                 160

Leu Lys Leu Ser Ala Asp Gln Val Ala Leu Val Tyr Ser Thr Leu Gly
                165                 170                 175

Leu Cys Leu Cys Ala Val Leu Cys Cys Phe Leu Val Ala Val Ala Cys
            180                 185                 190

Phe Leu Lys Lys Arg Gly Asp Pro Cys Ser Cys Gln Pro Arg Ser Arg
        195                 200                 205

Pro Arg Gln Ser Pro Ala Lys Ser Ser Gln Asp His Ala Met Glu Ala
    210                 215                 220

Gly Ser Pro Val Ser Thr Ser Pro Glu Pro Val Glu Thr Cys Ser Phe
225                 230                 235                 240

Cys Phe Pro Glu Cys Arg Ala Pro Thr Gln Glu Ser Ala Val Thr Pro
                245                 250                 255

Gly Thr Pro Asp Pro Thr Cys Ala Gly Arg Trp Gly Cys His Thr Arg
            260                 265                 270

Thr Thr Val Leu Gln Pro Cys Pro His Ile Pro Asp Ser Gly Leu Gly
        275                 280                 285

Ile Val Cys Val Pro Ala Gln Glu Gly Gly Pro Gly Ala
    290                 295                 300


<210> 13
<211> 10
<212> PRT
<213> Homo sapiens

<400> 13
Met Gly Val Ser Gly Arg Ser Arg Pro Ala
1               5                   10


<210> 14
<211> 1304
<212> DNA
<213> Homo sapiens

<220>
<221> CDS
<222> (42)..(1253)

<400> 14
taatacgact cactataggg agacccaagc ttaatcaaaa c atg gct atc atc tac 56
ctc atc ctc ctg ttc acc gct gtg cgg ggc ctc gac atg agt ggc ctg    104
ggc cgg agc agg cga ggt ggc cgg agc cgt gtg gac cag gag gag cgc 152
ttt cca cag ggc ctg tgg acg ggg gtg gct atg aga tcc tgc ccc gaa 200
gag cag tac tgg gat cct ctg ctg ggt acc tgc atg tcc tgc aaa acc 248
att tgc aac cat cag agc cag cgc acc tgt gca gcc ttc tgc agg tca 296
ctc agc tgc cgc aag gag caa ggc aag ttc tat gac cat ctc ctg agg 344
gac tgc atc agc tgt gcc tcc atc tgt gga cag cac cct aag caa tgt 392
gca tac ttc tgt gag aac aag ctc agg agc cca gtg aac ctt cca cca 440
gag ctc agg aga cag cgg agt gga gaa gtt gaa aac aat tca gac aac 488
tcg gga agg tac caa gga ttg gag cac aga ggc tca gaa gca agt cca 536
gct ctc ccg ggg ctg aag ctg agt gca gat cag gtc gac aaa act cac 584
aca tgc cca ccg tgc cca gca cct gaa ctc ctg ggg gga ccg tca gtc 632
ttc ctc ttc ccc cca aaa ccc aag gac acc ctc atg atc tcc cgg acc 680
cct gag gtc aca tgc gtg gtg gtg gac gtg agc cac gaa gac cct gag 728
gtc aag ttc aac tgg tac gtg gac ggc gtg gag gtg cat aat gcc aag 776
aca aag ccg cgg gag gag cag tac aac agc acg tac cgt gtg gtc agc 824
gtc ctc acc gtc ctg cac cag gac tgg ctg aat ggc aag gag tac aag 872
tgc aag gtc tcc aac aaa gcc ctc cca gcc ccc atc gag aaa acc atc 920
tcc aaa gcc aaa ggg cag ccc cga gaa cca cag gtg tac acc ctg ccc 968
cca tcc cgg gat gag ctg acc aag aac cag gtc agc ctg acc tgc ctg 1016
gtc aaa ggc ttc tat ccc agc gac atc gcc gtg gag tgg gag agc aat 1064
ggg cag ccg gag aac aac tac aag acc acg cct ccc gtg ttg gac tcc 1112
gac ggc tcc ttc ttc ctc tac agc aag ctc acc gtg gac aag agc agg 1160
tgg cag cag ggg aac gtc ttc tca tgc tcc gtg atg cat gag gct ctg 1208
cac aac cac tac acg cag aag agc ctc tcc ctg tct ccg ggt aaa 1253
tgagtgcgcg cggccgctct agagggccct attctatagt gtcacctaaa t 1304

<210> 15
<211> 404
<212> PRT
<213> Homo sapiens

<400> 15

<210> 16
<211> 546
<212> DNA
<213> Mus musculus

<220>
<221> CDS
<222> (16)..(543)

<400> 16
aagcttaatc aaaac atg gct atc atc tac ctc atc ctc ctg ttc acc gct   51
                 Met Ala Ile Ile Tyr Leu Ile Leu Leu Phe Thr Ala
                 1               5                   10

gtg cgg ggc gat tac aaa gac gat gac gat aaa gga ccc gga cag gtg   99
Val Arg Gly Asp Tyr Lys Asp Asp Asp Asp Lys Gly Pro Gly Gln Val
        15                  20                  25

cag ctg cag gca gta ctc acc cag aag cac aag aag aag cac tca gtc   147
Gln Leu Gln Ala Val Leu Thr Gln Lys His Lys Lys Lys His Ser Val
    30                  35                  40

ctg cat ctt gtt cca gtt aac att acc tcc aag gac tct gac gtg aca     195
Leu His Leu Val Pro Val Asn Ile Thr Ser Lys Asp Ser Asp Val Thr
 45                 50                  55                  60

gag gtg atg tgg caa cca gta ctt agg cgt ggg aga ggc ctg gag gcc     243
Glu Val Met Trp Gln Pro Val Leu Arg Arg Gly Arg Gly Leu Glu Ala
                65                  70                  75

cag gga gac att gta cga gtc tgg gac act gga att tat ctg ctc tat     291
Gln Gly Asp Ile Val Arg Val Trp Asp Thr Gly Ile Tyr Leu Leu Tyr
            80                  85                  90

agt cag gtc ctg ttt cat gat gtg act ttc aca atg ggt cag gtg gta     339
Ser Gln Val Leu Phe His Asp Val Thr Phe Thr Met Gly Gln Val Val
        95                  100                 105

tct cgg gaa gga caa ggg aga aga gaa act cta ttc cga tgt atc aga     387
Ser Arg Glu Gly Gln Gly Arg Arg Glu Thr Leu Phe Arg Cys Ile Arg
    110                 115                 120

agt atg cct tct gat cct gac cgt gcc tac aat agc tgc tac agt gca     435
Ser Met Pro Ser Asp Pro Asp Arg Ala Tyr Asn Ser Cys Tyr Ser Ala
125                 130                 135                 140

ggt gtc ttt cat tta cat caa ggg gat att atc act gtc aaa att cca     483
Gly Val Phe His Leu His Gln Gly Asp Ile Ile Thr Val Lys Ile Pro
                145                 150                 155

cgg gca aac gca aaa ctt agc ctt tct ccg cat gga aca ttc ctg ggg     531
Arg Ala Asn Ala Lys Leu Ser Leu Ser Pro His Gly Thr Phe Leu Gly
            160                 165                 170

ttt gtg aaa cta tga                                                 546
Phe Val Lys Leu
        175


<210> 17
<211> 176
<212> PRT
<213> Mus musculus

<400> 17
Met Ala Ile Ile Tyr Leu Ile Leu Leu Phe Thr Ala Val Arg Gly Asp
 1               5                  10                  15

Tyr Lys Asp Asp Asp Asp Lys Gly Pro Gly Gln Val Gln Leu Gln Ala
            20                  25                  30

Val Leu Thr Gln Lys His Lys Lys Lys His Ser Val Leu His Leu Val
        35                  40                  45

Pro Val Asn Ile Thr Ser Lys Asp Ser Asp Val Thr Glu Val Met Trp
    50                  55                  60

Gln Pro Val Leu Arg Arg Gly Arg Gly Leu Glu Ala Gln Gly Asp Ile
65                  70                  75                  80

Val Arg Val Trp Asp Thr Gly Ile Tyr Leu Leu Tyr Ser Gln Val Leu
                85                  90                  95

Phe His Asp Val Thr Phe Thr Met Gly Gln Val Val Ser Arg Glu Gly
            100                 105                 110

Gln Gly Arg Arg Glu Thr Leu Phe Arg Cys Ile Arg Ser Met Pro Ser
        115                 120                 125

Asp Pro Asp Arg Ala Tyr Asn Ser Cys Tyr Ser Ala Gly Val Phe His

130                 135                 140

Leu His Gln Gly Asp Ile Ile Thr Val Lys Ile Pro Arg Ala Asn Ala
145                 150                 155                 160

Lys Leu Ser Leu Ser Pro His Gly Thr Phe Leu Gly Phe Val Lys Leu
                165                 170                 175